(19) Europäisches Patentamt
European Patent Office
Office européen des brevets

(11) EP 0 457 723 B1

(12) **EUROPÄISCHE PATENTSCHRIFT**

(45) Veröffentlichungstag und Bekanntmachung des Hinweises auf die Patenterteilung:
**22.01.1997 Patentblatt 1997/04**

(21) Anmeldenummer: **91810348.2**

(22) Anmeldetag: **07.05.1991**

(51) Int Cl.6: **G03C 7/392**, C07C 69/732, C07C 69/88, C07C 69/675, C07C 69/36, C07C 69/22, C07C 309/73

(54) **Verfahren zum Stabilisieren von Magentakupplern und den entsprechenden Bildfarbstoffen in photographischen Materialien**

Method for the stabilization of magenta couplers and the resulting dye-images in photographic materials

Méthode pour la stabilisation de coupleurs magenta et des images de colorants résultantes dans des produits photographiques

(84) Benannte Vertragsstaaten:
**DE FR GB IT**

(30) Priorität: **16.05.1990 CH 1653/90**

(43) Veröffentlichungstag der Anmeldung:
**21.11.1991 Patentblatt 1991/47**

(73) Patentinhaber: **Ciba SC Holding AG**
**4057 Basel (CH)**

(72) Erfinder:
- **Leppard, David G., Dr.**
  **CH-1723 Marly (CH)**
- **Steinberg, David H., Dr.**
  **Bronx, N.Y. 10469 (US)**
- **Dubas, Henri, Dr.**
  **CH-1723 Marly (CH)**

(56) Entgegenhaltungen:
**EP-A- 0 154 336** **DE-A- 3 605 279**
**GB-A- 2 022 274**

- **MATERIE PLASTICHE ELASTOMERI Januar 1975, Italy Seiten 41 - 44; Y.I.TEMCHIN et**
- **AL.: "Dependance of the efficiency of photostabilizers for polypropylene upon their chemical structure"**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 457 723 B1

## Beschreibung

Die vorliegende Erfindung betrifft ein Verfahren zum Stabilisieren von Magentakupplern und den entsprechenden Bildfarbstoffen in photographischen Materialien unter Verwendung von Benzoylgruppen enthaltenden Verbindungen.

Bildfarbstoffe in praktisch allen photographischen Bildern unterliegen im Laufe der Zeit durch Einwirkung von Luftsauerstoff, Feuchtigkeit und Wärme sowie durch Licht Veränderungen, die sich in Farbverschiebungen und Verlusten an Kontrast und Farbdichte äussern. Dies trifft besonders für die nach der chromogenen Entwicklung erhaltenen Bilder zu und speziell für solche Bilder, die auch nach der Verarbeitung noch Kupplermoleküle enthalten. In diesen Bildern beobachtet man nicht nur ein Ausbleichen der Bildfarbstoffe, sondern auch die unerwünschte Vergilbung von Bildweissen und die Farbstoffbildung durch Reaktionen dieser Kupplermoleküle z.B. mit Bildfarbstoff und mit sich selbst. Die verschiedenen Geschwindigkeiten solcher Reaktionen bei Gelb-, Cyan- und Magentakupplern führen in den Bildern zu den genannten Farbverschiebungen und Kontrastverlusten.

Die unerwünschte Vergilbung heller Bildstellen und Farbbildungsreaktion der Kupplermoleküle verläuft sowohl am Licht als auch im Dunkeln. Somit kommt der Stabilisierung photographischer Bilder eine besondere Bedeutung zu.

Besonders die hohe Dunkelstabilität von photographischen Bildern wird auch von Einrichtungen wie Museen, Archiven, Agenturen und Galerien gefordert, die an der Erhaltung der ursprünglichen Bildfarbstoffe interessiert sein müssen.

In diesem Zusammenhang schlägt die japanische Offenlegungsschrift 52/082219 zur Verbesserung der Dunkelstabilität photographischer Bilder die Verwendung von Polyvinylimidazolen vor. Für denselben Zweck werden substituierte Phosphorsäure-(4-hydroxy)anilide in der japanischen Offenlegungsschrift 53/108428 eingesetzt. Brenzkatechindiäthyläther bzw. Dibenzoxaphosphorine sind als Stabilisatoren für die Dunkellagerung photographischer Bilder in der japanischen Offenlegungsschrift 57/204036 bzw. in US 4,661,440 beschrieben.

DE-A-3 605 279 beschreibt lichtempfindliches photographisches Silberhalogenidaufzeichnungsmaterial, das als Purpurrotkuppler ein Pyrazoloazol-Derivat und einen Metallkomplex sowie mindestens ein Phenol-Derivat enthält.

Es wurde nun gefunden, dass gewisse Benzoylgruppen enthaltende Verbindungen photographischen Materialien durch Stabilisierung der darin enthaltenen Magentakuppler eine gute Dunkelstabilität verleihen können. Unter "photographischen Materialien" sind im folgenden sowohl unbelichtete, Magentakuppler enthaltende Materialien als auch daraus hergestellte photographische Bilder zu verstehen, da die erfindungsgemäss verwendeten Verbindungen schon in den Schichten dieser unbelichteten Materialien vorliegen und ihre stabilisierende Wirkung bereits darin entfalten.

Gegenstand der vorliegenden Erfindung ist somit ein Verfahren zum Stabilisieren von Magentakupplern und entsprechenden Bildfarbstoffen in photographischen Materialien, dadurch gekennzeichnet, dass in die Magentakuppler enthaltende Schicht oder in eine an diese angrenzende Schicht mindestens eine Verbindung der Formel

(1) 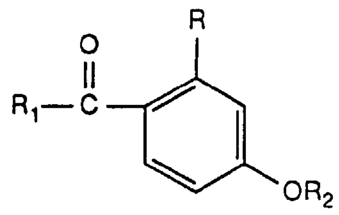

eingearbeitet wird, worin
R Wasserstoff oder Hydroxyl,
$R_1$ Alkyl oder Alkoxy mit je 1 bis 18 Kohlenstoffatomen, $-NR_4R_5$, worin $R_4$ und $R_5$ unabhängig voneinander Wasserstoff oder Alkyl mit 1 bis 18 Kohlenstoffatomen sind, oder $R_1$ ein Rest der Formel

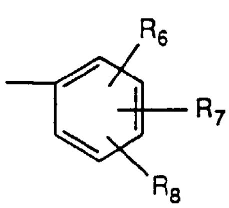 ,

ist worin $R_6$, $R_7$ und $R_8$ unabhängig voneinander Wasserstoff, Hydroxyl oder Alkyl mit je 1 bis 8 Kohlenstoffatomen

sind, oder $R_1$ ein Rest der Formel

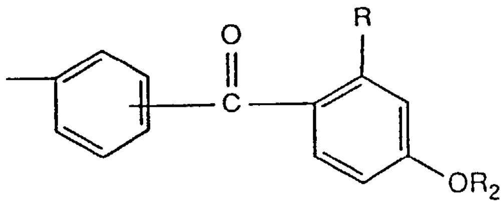

ist, worin R die angegebene Bedeutung hat, und
$R_2$ -$COR_{11}$ oder -$SO_2R_{11}$, worin $R_{11}$ Alkyl mit 1 bis 18 Kohlenstoffatomen, Alkenyl mit 2 bis 18 Kohlenstoffatomen, Phenylalkyl mit 1 bis 4 Kohlenstoffatomen im Alkylteil oder ein Rest der Formel

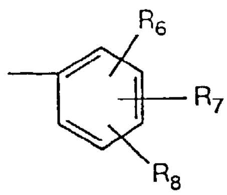

,

ist, worin $R_6$, $R_7$ und $R_8$ die angegebenen Bedeutungen haben, oder $R_2$ ein Rest der Formel

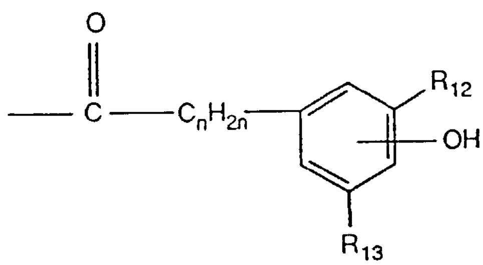

,

oder

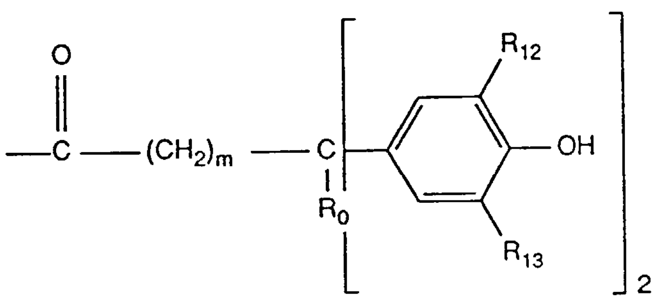

ist, worin m 1 bis 6 und $R_0$ Wasserstoff oder Methyl ist, und n 0 bis 14 ist, und $R_{12}$ und $R_{13}$ unabhängig voneinander Wasserstoff, Alkyl mit je 1 bis 8 Kohlenstoffatomen, Cyclopentyl, Cyclohexyl, Alkylphenyl mit 1 bis 8 Kohlenstoffatmen im Alkylteil oder Phenyl sind.

Gegenstand der vorliegenden Erfindung sind ferner photographische Materialien, worin die Verbindung der Formel (1) in einer Magentakuppler enthaltenden Schicht oder in einer an diese Schicht angrenzenden Schicht enthalten ist,

sowie die neuen Verbindungen der Formel (1a).

In den Verbindungen der Formel (1) kann der in Orthostellung zur Carbonylgruppe stehende Substituent R Wasserstoff oder Hydroxyl bedeuten.

$R_1$ kann Alkyl mit 1 bis 18 Kohlenstoffatomen bedeuten, also beispielsweise Methyl, Ethyl, Propyl, Butyl, Pentyl, Hexyl, Heptyl, Octyl, Nonyl, Undecyl, Dodecyl, Hexadecyl und Octadecyl sowie entsprechende verzweigte Isomere. Des weiteren kann $R_1$ Alkoxy mit 1 bis 18 Kohlenstoffatomen sein. Geeignete Beispiele für Alkoxyreste $R_1$ sind der vorstehenden Aufzählung zu entnehmen. Des weiteren kann $R_1$ eine Aminogruppe der Formel $-NR_4R_5$ bedeuten, wobei $R_4$ und $R_5$ unabhängig voneinander neben Wasserstoff auch Alkyl mit 1 bis 18 Kohlenstoffatmen bedeuten. Geeignete Alkylreste sind oben aufgeführt $R_1$ ist bevorzugt Alkyl oder Alkoxy mit 1 bis 8 Kohlenstoffatomen. $R_1$ kann auch für eines der folgenden aromatischen Systeme der Formel

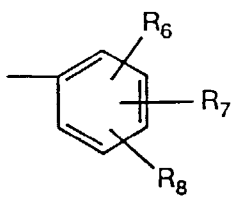

oder

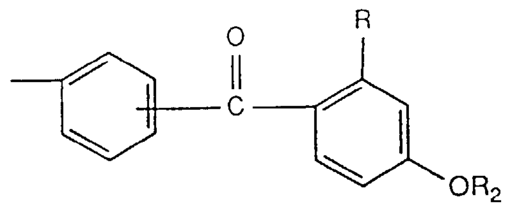

stehen. Hierin bedeuten die Substituenten $R_6$, $R_7$ und $R_8$ unabhängig voneinander Wasserstoff, Hydroxyl, Alkyl mit 1 bis 8 Kohlenstoffatomen wie Methyl, Propyl, Butyl, Hexyl und Octyl sowie entsprechende verzweigte Isomere. Der Substituent R hat die oben angegebene Bedeutung. Für den Substituenten $R_2$ kommen die Reste der Formel $-COR_{11}$, $-SO_2R_{11}$, $-COCO_2R_{10}$, $-CO_2R_{10}$,

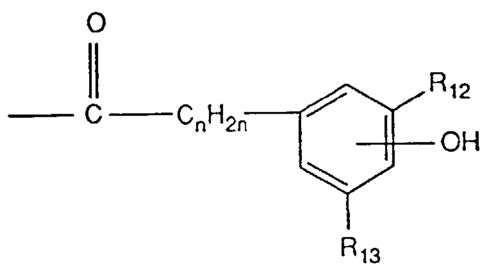

oder

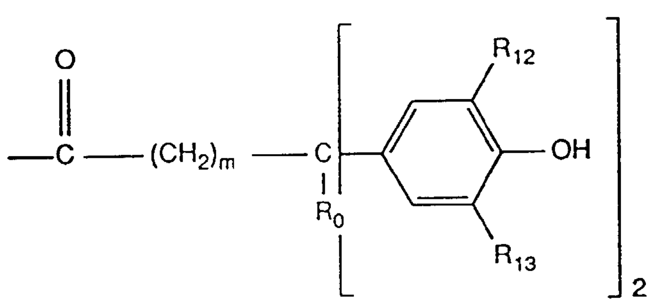

in Frage. $R_{11}$ bedeutet Alkyl bzw. Alkenyl mit 1 bzw. 2 bis 18 Kohlenstoffatomen (s.o.), ferner Phenylalkyl mit 1 bis 4 Kohlenstoffatomen im Alkylteil (s.o.) oder ein Rest der Formel

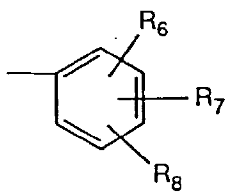

,

worin $R_6$, $R_7$ und $R_8$ die angegebenen Bedeutungen haben. Die Substituenten $R_{12}$ und $R_{13}$ bedeuten unabhängig voneinander neben Wasserstoff Alkyl mit je 1 bis 8 Kohlenstoffatome (s.o.), Cyclopentyl oder Cyclohexyl, ferner Alkylphenyl mit je 1 bis 8 Kohlenstoffatomen im Alkylteil (s.o.) oder Phenyl. $R_0$ ist Wasserstoff oder Methyl, n eine ganze Zahl von 0 bis 14, m von 1 bis 6.

In einer Gruppe bevorzugter Verbindungen der Formel (1) ist $R_1$ Alkyl oder Alkoxy mit je 1 bis 18 Kohlenstoffatomen oder ein Rest der Formel

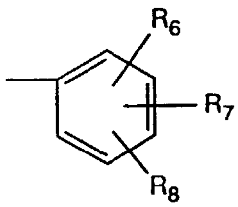

oder

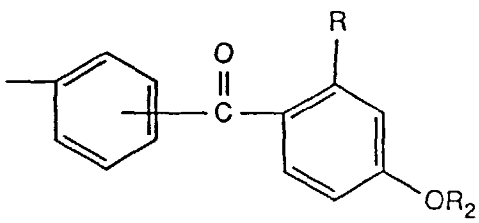

,

worin R, $R_2$, $R_6$, $R_7$ und $R_8$ die angegebene Bedeutung haben, wobei $R_1$ insbesondere Alkyl oder Alkoxy mit je 1 bis 8 Kohlenstoffatomen oder ein Rest der Formel

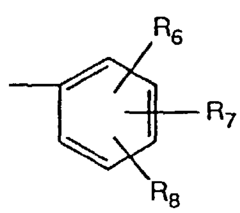

ist, worin $R_6$, $R_7$ und $R_8$ unabhängig voneinander Wasserstoff, Hydroxyl oder Alkyl mit 1 bis 4 Kohlenstoffatomen sind, R Hydroxyl und $R_2$ $-COR_{11}$, worin $R_{11}$ Alkyl mit 1 bis 18 Kohlenstoffatomen ist, oder ein Rest der Formel

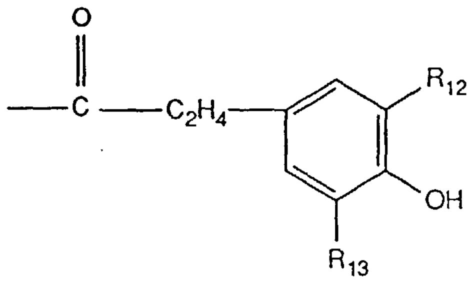

ist, worin $R_{12}$ und $R_{13}$ unabhängig voneinander Wasserstoff oder Alkyl mit 1 bis 4 Kohlenstoffatomen sind.

Hiervon besonders bevorzugt sind solche Verbindungen, worin $R_1$ Alkyl oder Alkoxy mit je 1 bis 4 Kohlenstoffatomen oder ein Rest der Formel

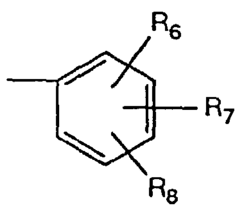

ist, worin $R_6$, $R_7$ und $R_8$ unabhängig voneinander Wasserstoff, Hydroxyl oder Alkyl mit 1 bis 4 Kohlenstoffatomen sind, und $R_2$ $-COR_{11}$, worin $R_{11}$ Alkyl mit 12 bis 18 Kohlenstoffatomen ist, oder ein Rest der Formel

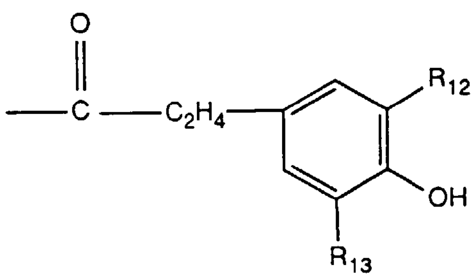

ist, worin $R_{12}$ und $R_{13}$ Alkyl mit je 1 bis 4 Kohlenstoffatomen sind.

Als spezifische Beispiele von Verbindungen der Formel (1) seien die Verbindungen der Formeln (2) bis (33) aufgeführt.

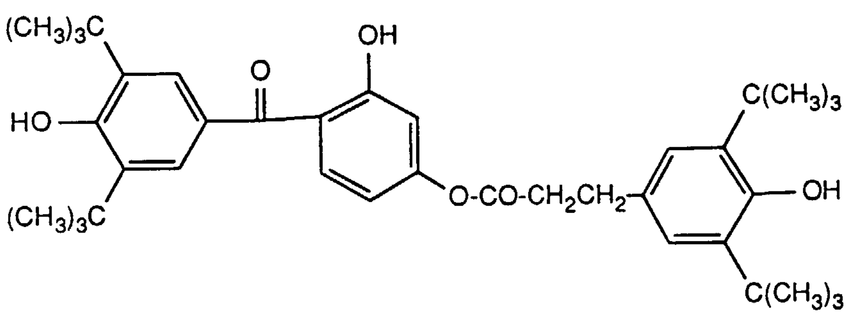

(2)

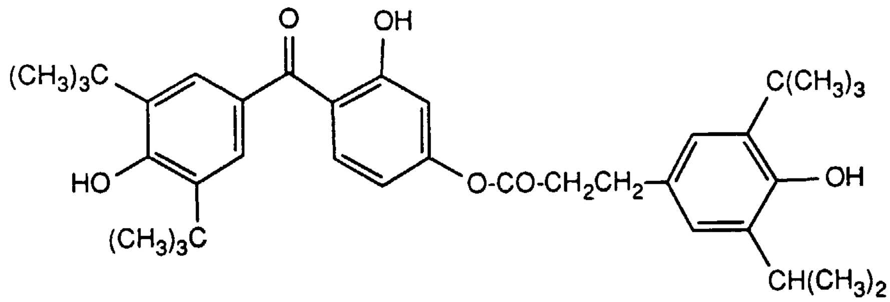

(3)

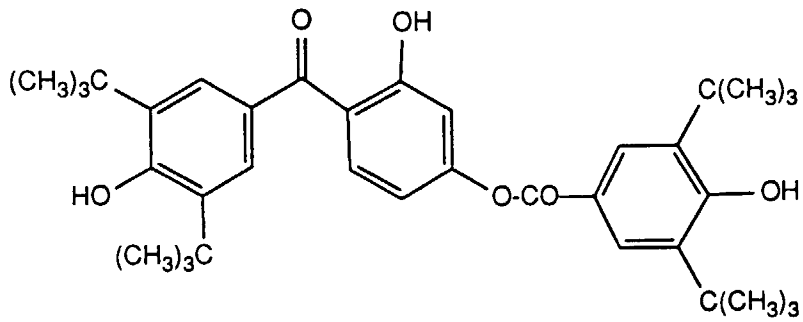

(4)

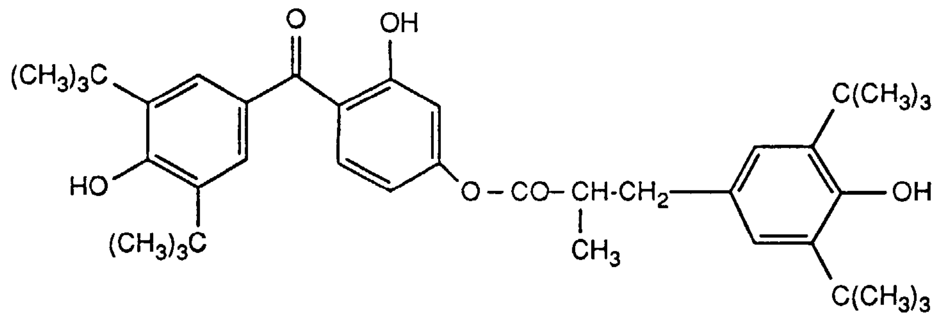

(5)

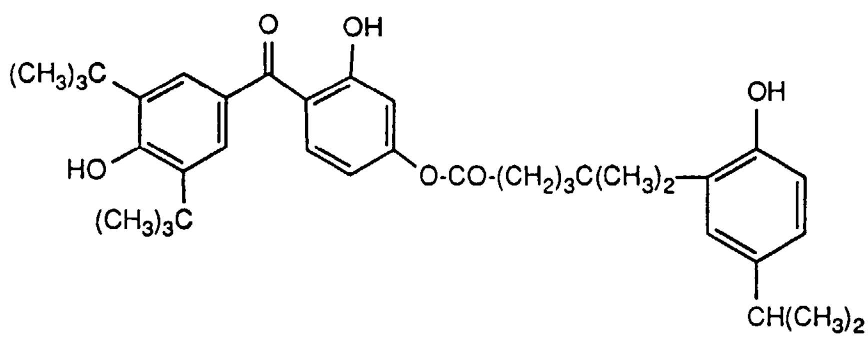

(6)

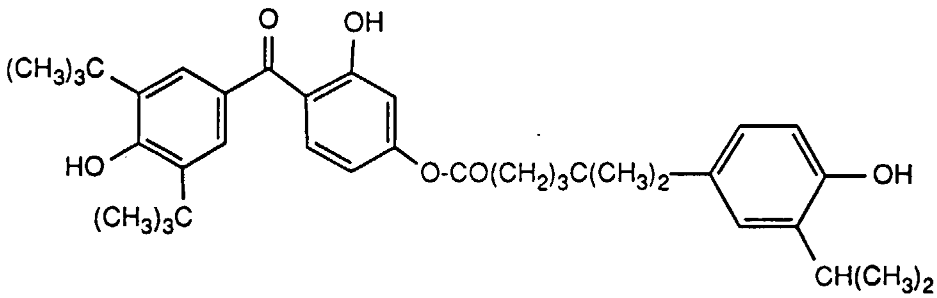

(7)

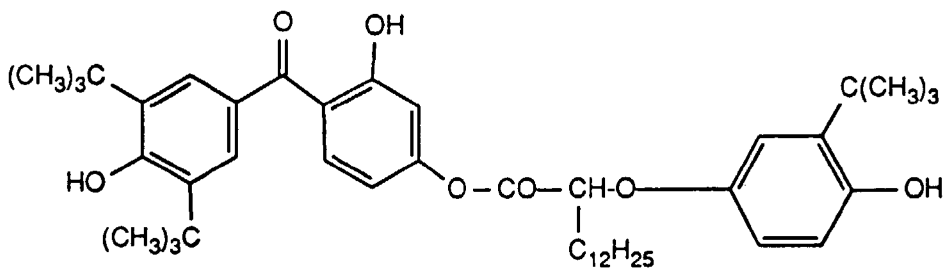

(8)

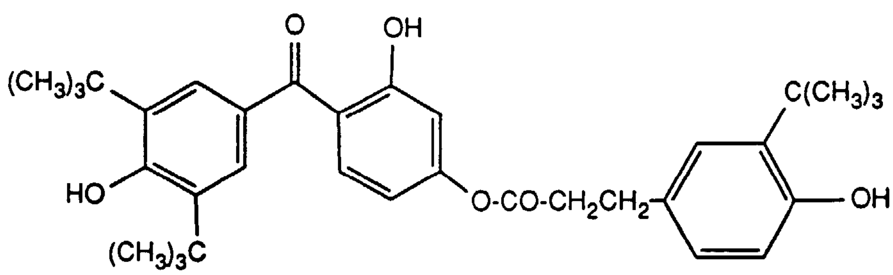

(9)

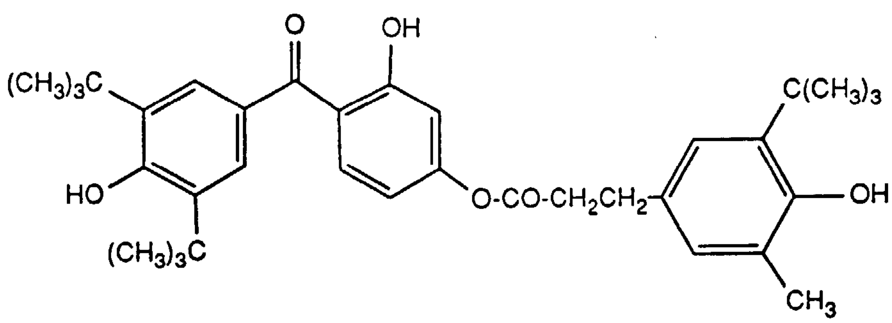

(10)

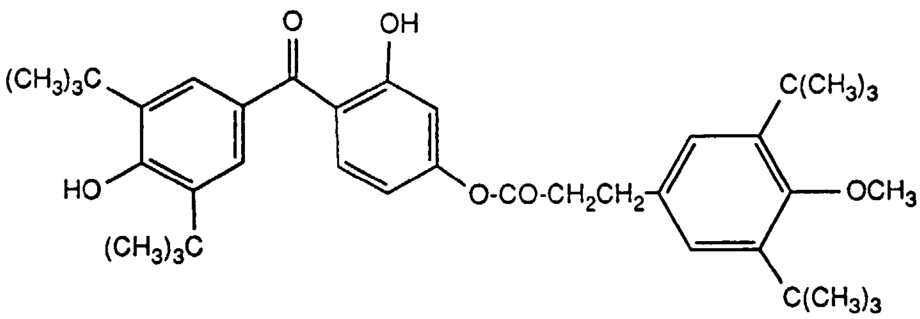

(11)

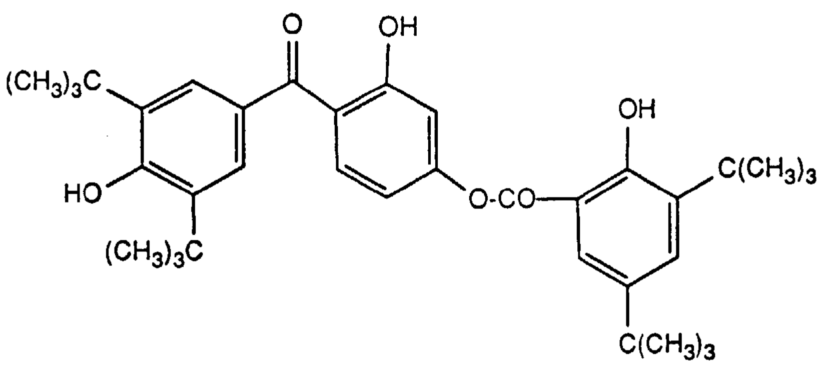

(12)

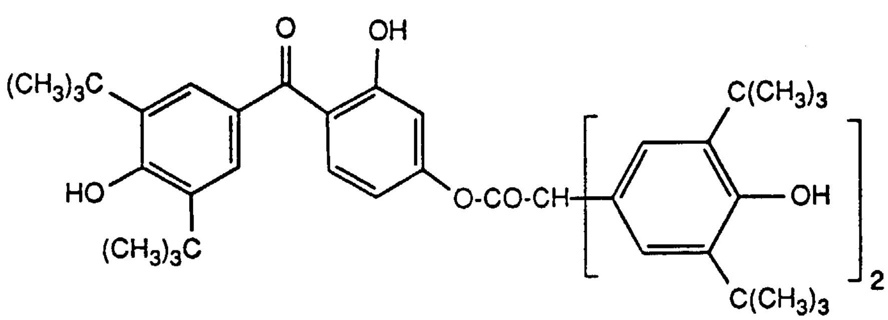

(13)

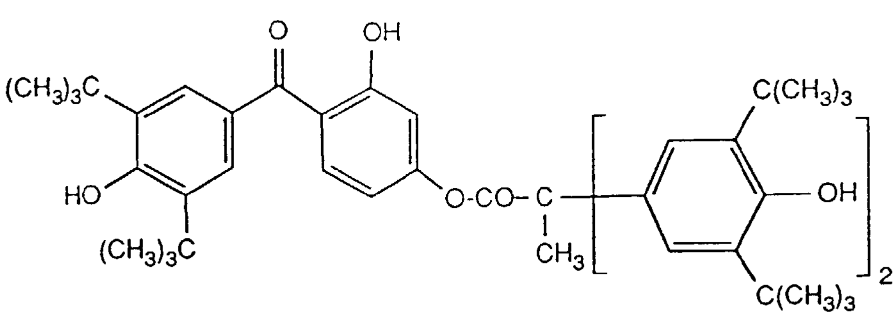

(14)

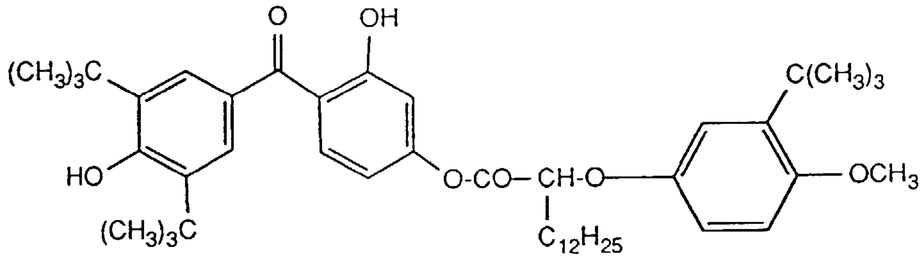

(15)

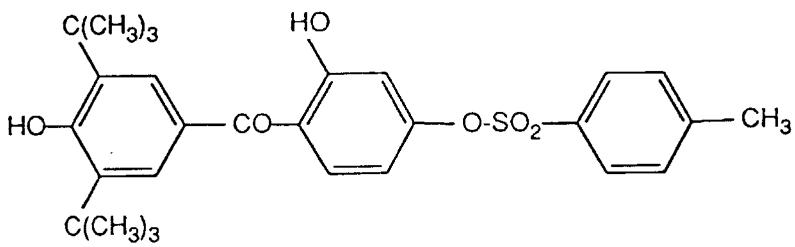

(16)

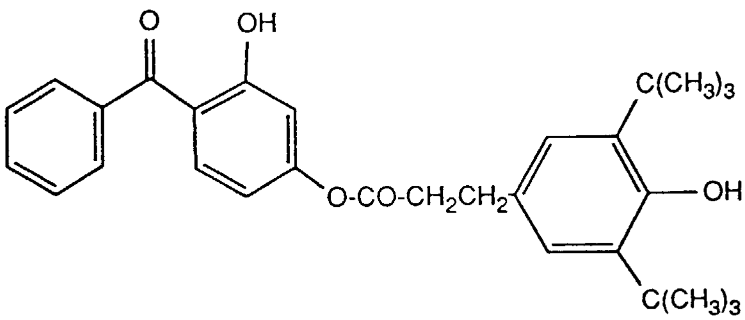

(18)

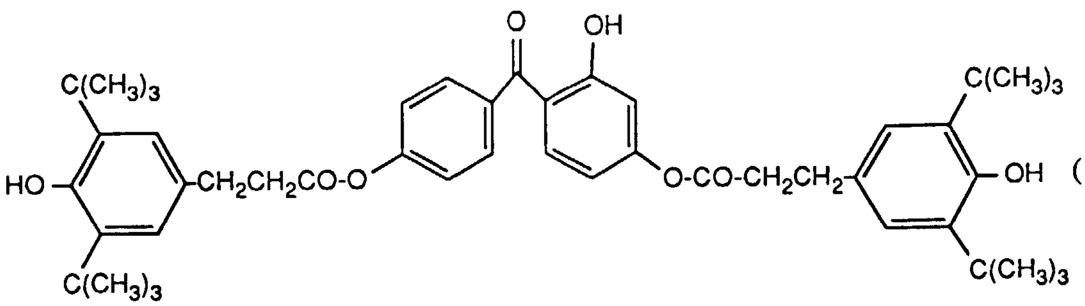

(19)

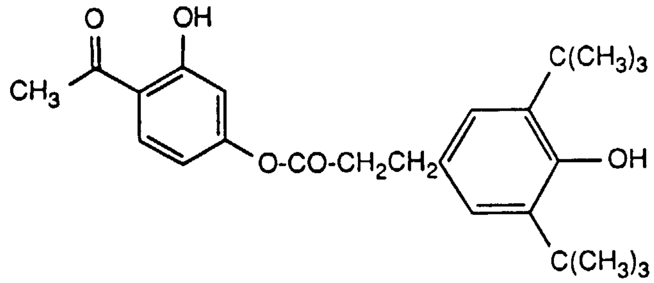

(20)

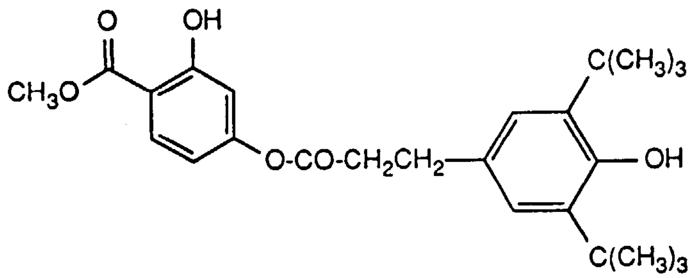

(21)

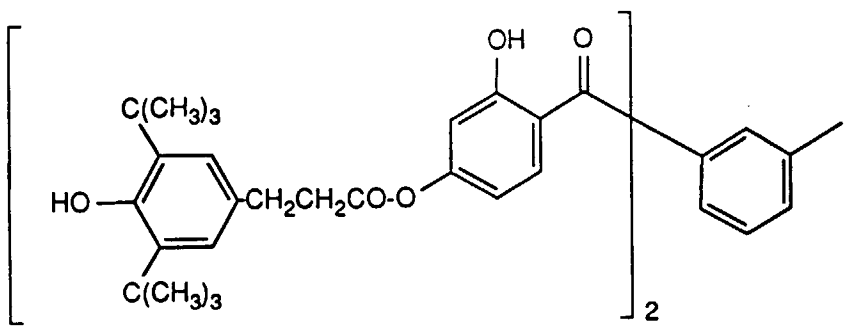

(22)

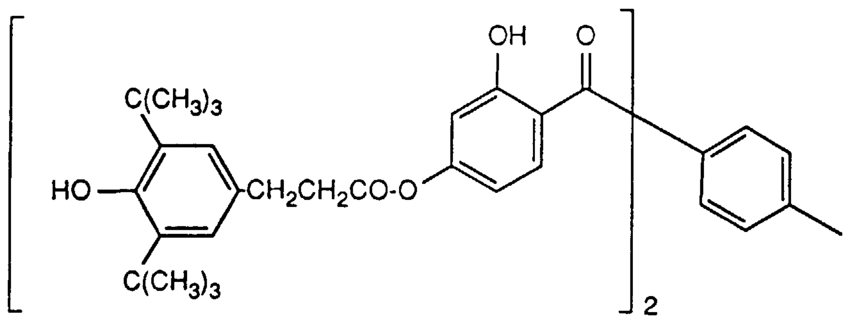

(23)

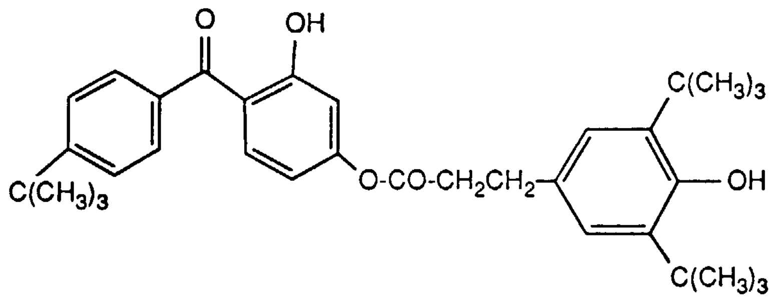

(24)

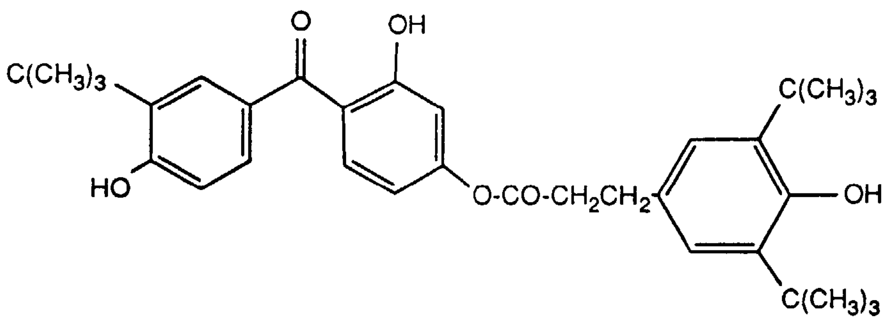

(25)

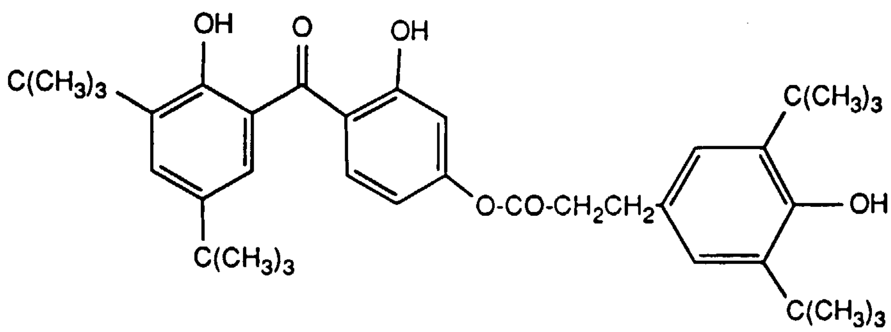

(26)

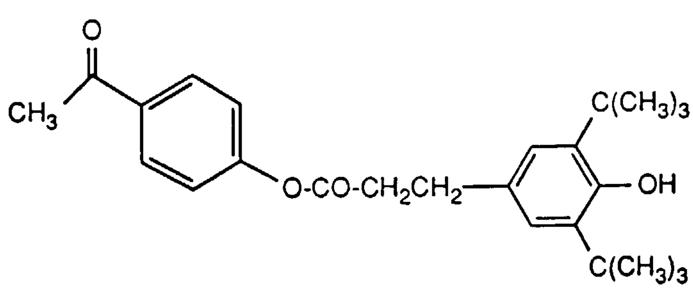

(27)

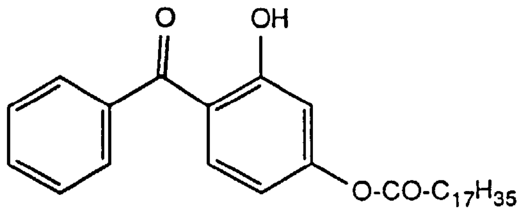

(28)

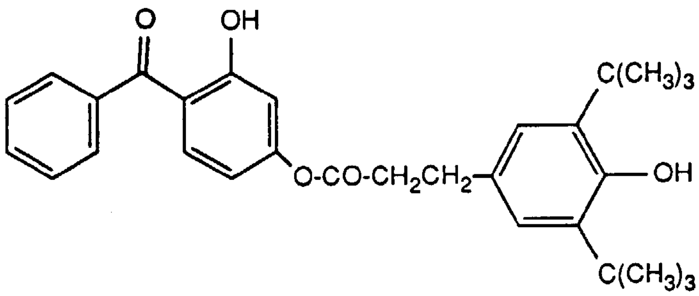

(29)

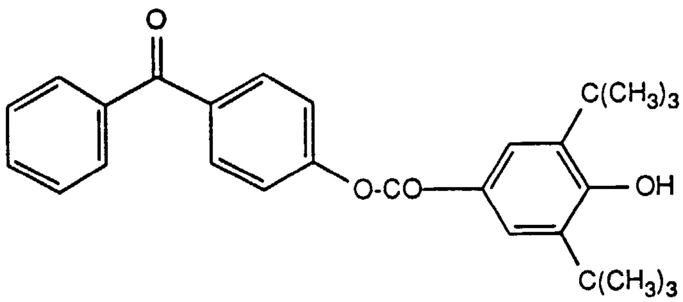

(30)

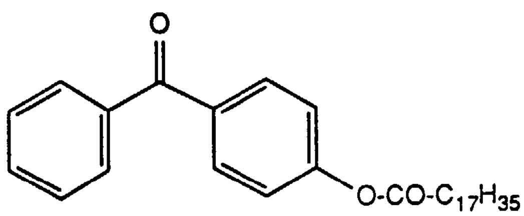

(31)

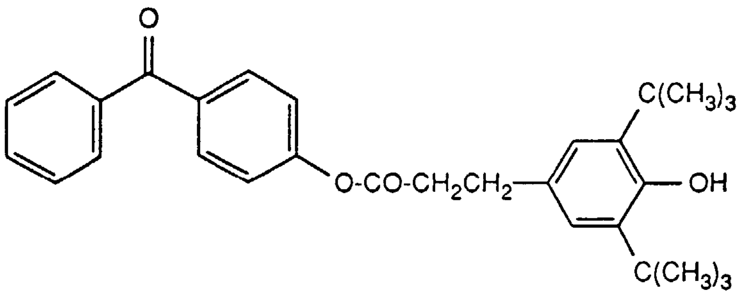

(32)

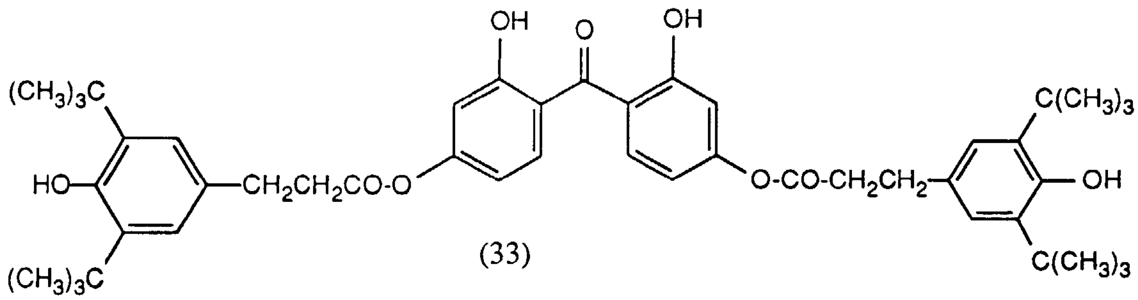

(33)

Mit den Verbindungen der Formel (1) können praktisch alle Typen von Magentakuppler in photographischen Materialien stabilisiert werden. Diese Magentakuppler können z.B. einfache 1-Aryl-5-pyrazolone sein oder mit 5-gliedrigen Heteroringen kondensierte Pyrazolderivate wie z.B. Imidazopyrazole, Pyrazolotriazole oder Pyrazolotetrazole.

Eine Gruppe von Magentakupplern sind 5-Pyrazolone der Formel

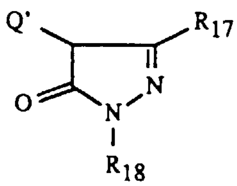

(A),

wie sie in der Britischen Patentschrift 2,003,473 beschrieben sind. Darin ist $R_{17}$ Wasserstoff, Alkyl, Aryl, Alkenyl oder eine heterocyclische Gruppe. $R_{18}$ ist Wasserstoff, Alkyl, Aryl, eine heterocyclische Gruppe, eine Estergruppe, Alkoxygruppe, Alkylthiogruppe, Carboxylgruppe, Arylaminogruppe, Acylaminogruppe, (Thio)-harnstoffgruppe, (Thio)-carbamoylgruppe, Guanidinogruppe oder Sulfonamidogruppe. Q' ist eine Abgangsgruppe.

Typische Beispiele für diesen Typ von Magentakupplern sind Verbindungen der Formel

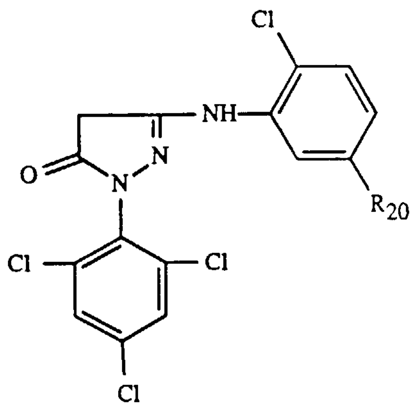

(B),

worin $R_{20}$ Wasserstoff, Alkyl, Acylamino, Carbamoyl, Sulfamoyl, Sulfonamid, Alkoxycarbonyl, Acyloxy oder eine Urethangruppe ist.

Weitere Beispiele solcher Vieräquivalent-Magentakuppler sind zu finden in den US-A 2,983,608, 3,061,432, 3,062,653, 3,127,269, 3,152,896, 3,311,476, 3,419,391, 3,519,429, 3,558,319, 3,582,322, 3,615,506, 3,684,514, 3,834,908, 3,888,680, 3,891,445, 3,907,571, 3,928,044, 3,930,861, 3,930,866, 3,933,500.

Wenn Q′ nicht Wasserstoff ist sondern eine Gruppe, die bei der Reaktion mit dem oxidierten Entwickler eliminiert wird, so handelt es sich um Zweiäquivalent-Magentakuppler, welche beispielsweise in US-A 3,006,579, 3,419,391, 3,311,476, 3,432,521, 3,214,437, 4,032,346, 3,701,783, 4,351,897, 3,227,554, in den EP-A-133,503, DE-A-2,944,601, JP-A-78/34044, 74/53435, 74/53436, 75/53372 und 75/122935 beschrieben sind.

Ueber ein zweiwertiges Q′ können 2-Pyrazolonringe verknüpft werden und man erhält dann sogenannte Bis-Kuppler. Solche sind z.B. beschrieben in den US-A-2,632,702, US-A-2,618,864, GB-A-968,461, GB-A-786,859, JP-A-76/37646, 59/4086, 69/16110, 69/26589, 74/37854 und 74/29638.

Weitere Typen von Magentakupplern, die von den Verbindungen der Formel (1) besonders gut stabilisiert werden können, sind Pyrazoloazol-Magentakuppler, wie z.B Pyrazolo-tetrazole, beschrieben in der JP-A-85/33552; Pyrazolo-pyrazole in der JP-A-85/43,695; Pyrazolo-imidazole in den JP-A-85/35732, JP-A-86/18949 und US-A-4,500,630; Pyrazolo-triazole in den JP-A-85/186,567, JP-A-86/47957, JP-A-85/215,687, JP-A-85/197,688, JP-A-85/172,982, EP-A-119,860, EP-A-173,256, EP-A-178,789, EP-A-178,788 und in Research Disclosure 84/24,624.

Weitere Pyrazoloazol-Magentakuppler sind beschrieben in: JP-A-86/28,947, JP-A-85/140,241, JP-A-85/262,160, JP-A-85/213,937, EP-A-177,765, EP-A-176,804, EP-A-170,164, EP-A-164,130, EP-A-178,794, DE-A-3,516,996, DE-A-3,508,766 und Research Disclosure 81/20919, 84/24531 und 85/25758.

Pyrazoloazol-Magentakuppler können durch die Formel

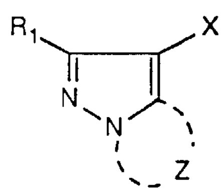

(C)

wiedergegeben werden, worin $R_1$ Wasserstoff oder ein Substituent, X Wasserstoff oder eine Abgangsgruppe ist, und Z die nichtmetallischen Atome darstellt, um einen 5-gliedrigen Ring mit 2 oder 3 Stickstoffatomen zu bilden. Vorzugsweise liegen diese Magentakuppler in den folgenden Strukturen vor:

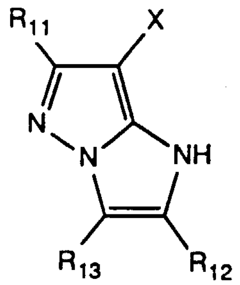
(C-I)

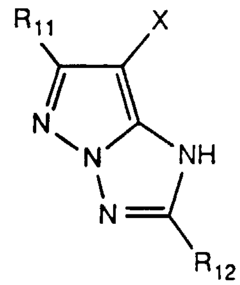
(C-II)

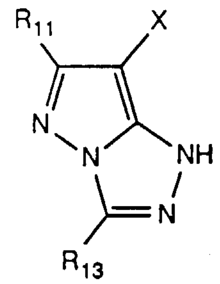
(C-III)

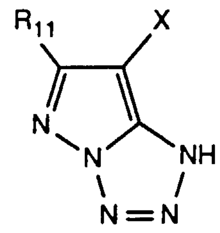
(C-IV)

Der Substituent $R_{11}$ in diesen Formeln bedeutet beispielsweise Wasserstoff, Halogen, Alkyl, Aryl, eine heterocyclische Gruppe, Cyano, Hydroxy, Nitro, Carboxyl, Amino, Alkoxy, Aryloxy, Acylamino, Alkylamino, Anilino, Ureido, Sulfamoylamino, Alkylthio, Arylthio, Alkoxycarbonylamino, Sulfonamido, Carbamoyl, Sulfamoyl, Sulfonyl, Alkoxycarbonyl, Azo, Acyloxy, Carbamoyloxy, Silyloxy, Aryloxycarbonylamino, Imino, Sulfinyl, Phosphonyl, Aryloxycarbonyl, Acyl or Azolyl.

Ist $R_{11}$ ein zweiwertiger Rest, so resultiert die entsprechende Bis-Form.

Insbesondere kann $R_{11}$ bedeuten: Wasserstoff, Halogen, z.B. Chlor und Brom; Alkyl z.B. mit 1 bis 32 Kohlenstoffatomen, Aralkyl, Alkenyl, Alkinyl, Cycloalkyl oder Cycloalkenyl und vorzugsweise Methyl, Ethyl, Propyl, Isopropyl, t-Butyl, Tridecyl, 2-Methansulfonylethyl, 3-(3-Pentadecylphenoxy)propyl, 3-(4-(2-(4-(4-(Hydroxyphenylsulfonyl)phenoxy)dodecanamido)phenyl)propyl, 2-Ethoxytridecyl, Trifluormethyl, Cyclopentyl, 3-(2,4-di-t-Amylphenoxy)propyl; Aryl, z.B. Phenyl, 4-t-Butylphenyl, 2,4-di-t-Amylphenyl, 4-Tetradecaneamidophenyl; eine heterocyclische Gruppe wie 2-Furyl, 2-Thienyl, 2-Pyrimidinyl, 2-Benzothiazolyl; Cyano; Hydroxyl; Nitro; Carboxy; Amino; Alkoxy wie Methoxy, Ethoxy, 2-Methoxyethoxy, 2-Dodecylethoxy, 2-Methansulfonylethoxy; Aryloxy wie Phenoxy, 2-Methylphenoxy, 4-t-Butylphenoxy, 3-Nitrophenoxy, 3-t-Butyloxy-carbamoylphenoxy, 3-Methoxycarbamoyl; Acylamino wie Acetoamido, Benzamido, Tetradecanamido, 2-(2,4-di-t-Amylphenoxy)butanamido, 4-(3-t-Butyl-4-hydroxyphenoxy)butanamido, 2-(4-(4-Hydroxyphenylsulfonyl)phenoxy)decanamido; Alkylamino wie Methylamino, Butylamino, Dodecylamino, Diethylamino, Methylbutylamino; Anilino wie Phenylamino, 2-Chloroanilino, 2-Chloro-5-tetradecanaminoanilino, 2-Chloro-5-dodecyloxycarbonylanilino, N-Acetylanilino, 2-Chloro-5-(alpha-(3-t-butyl-4-hydroxyphenoxy)dodecanamidoanilino; Ureido wie Phenylureido, Methylureido, N,N-Dibutyl-ureido; Sulfamoylamino wie N,N-Dipropylsulfamoylamino, N-Methyl-N-decylsulfamoylamino; Alkylthio wie Methylthio, Octylthio, Tetradecylthio, 2-Phenoxyethylthio, 3-Phenoxypropylthio, 3-(4-t-Butylphenoxy)propylthio; Arylthio wie Phenylthio, 2-Butoxy-5-t-octylphenylthio, 3-Pentadecylphenylthio, 2-Carboxyphenylthio, 4-Tetradecanamidophenylthio; Alkoxycarbonylamino wie Methoxycarbonylamino, Tetradecyloxycarbonylamino; Sulfonamido wie Methansulfonamido, p-Toluolsulfonamido, Octadecansulfonamido; Carbamoyl wie N-Ethylcarbamoyl, N,N-Dibutylcarbamoyl, N-(2-Dodecyloxyethyl)-carbamoyl, N-Methyl-N-dodecylcarbamoyl, N-(3-(2,4-di-t-Amylphenoxy)propyl)-carbamoyl; Sulfamoyl wie N-Ethylsulfamoyl, N,N-Dipropylsulfamoyl, N-(2-Dodecyloxyethyl)sulfamoyl, N-Ethyl-N-dodecylsulfamoyl, N-Ethyl-N-dodecylsulfamoyl, N,N-Diethylsulfamoyl; Sulfonyl wie Methansulfonyl, Octansulfonyl, Phenylsulfonyl, Toluolsulfonyl; Alkoxycarbonyl wie Methoxycarbonyl, Butoxycarbonyl, Dodecyloxycabonyl, Octadecyloxycarbonyl; eine heterocyclische Gruppe wie 1-Phenyltetrazole-5-oxy, 2-Tetrahydropyranyloxy; Azo wie Phenylazo, 4-Methoxyphenylazo, 4-Pivaloylaminophenylazo, 2-Hydroxy-4-propanoylphenylazo; Acyloxy wie Acetoxy; Carbamoyloxy wie N-Methylcarbamoyloxy, N-Phenylcarbamoyloxy; Silyloxy wie Trimethylsilyloxy, Dibutylmethylsilyloxy; Aryloxycarbonylamino wie Phenoxycarbonylamino; Imido wie N-Succinimido, N-Phthalimido, 3-Octadecenylsuccinimido; eine heterocyclische Gruppe wie Benzothiazolylthio, 2,4-Diphenoxy-1,3,5-triazole-6-thio, 2-Pyridylthio; Sulfinyl wie Dodecylsulfinyl, 3-Pentadecylphenylsulfinyl, 3-Phenoxypropylsulfinyl; Phosphonyl wie Phenoxyphosphonyl, Octyloxyphosphonyl, Phenylphosphonyl; Aryloxycarbonyl, wie Phenoxycarbonyl; Acyl, wie Acetyl, 3-Phenylpropanoyl, Benzoyl, 4-Dodecyloxybenzoyl; Azolyl, wie Imidazolyl, Pyrazolyl, 3-Chloro-pyrazol-1-yl.

Hiervon besonders bevorzugt sind Alkyl, Aryl, Alkoxy, Aryloxy, Alkylthio, Ureido, Urethan und Acylamino.

$R_{12}$ kann eine der Bedeutungen von $R_{11}$ besitzen und ist vorzugsweise Wasserstoff, Alkyl, Aryl, eine heterocyclische Gruppe, Alkoxycarbonyl, Carbamoyl, Sulfamoyl, Sulfinyl, Acyl oder Cyano.

$R_{13}$ kann ebenfalls eine der Bedeutungen von $R_{11}$ besitzen und ist vorzugsweise Wasserstoff, Alkyl, Aryl, eine heterocyclische Gruppe, Alkoxy, Aryloxy, Alkylthio, Arylthio, Alkoxycarbonyl, Carbamoyl oder Acyl.

X ist Wasserstoff oder eine Gruppe, die bei Reaktion mit dem Entwickleroxidationsprodukt abgespalten wird. Beispiele solcher Abgangsgruppen sind Halogen, Alkoxy, Aryloxy, Acyloxy, Alkyl- oder Arylsulfonyloxy, Acylamino, Alkyl- oder Arylsulfonamido, Alkoxycarbonyloxy, Aryloxycarbonyloxy, Alkyl- oder Arylthio, Carbamoylamino, Imido, Arylazo

usw. Diese Gruppen sind gegebenenfalls weiter substituiert

Vorzugsweise ist X Halogen wie Fluor, Chlor und Brom; Alkoxy wie Ethoxy, Dodecyloxy, Methoxyethylcarbamoylmethoxy, Carboxypropyloxy, Methylsulfonylethoxy, Ethoxycarbonylmethoxy; Aryloxy wie 4-Methylphenoxy, 4-Chlorophenoxy, 4-Methoxyphenoxy, 4-Carboxyphenoxy, 3-Ethoxycarboxyphenoxy, 3-Acetylaminophenoxy, 2-Carboxyphenoxy; Acetyloxy wie Acetoxy, Tetradecanoyloxy, Benzoyloxy; Alkyl- oder Arylsulfonyloxy wie Methansulfonyloxy, Tolylsulfonyloxy; Acylamino wie Dichloracetylamino, Heptafluorobutyrylamino; Alkyl- oder Arylsulfonamido wie Methansulfonamido, Trifluoromethansulfonamido, p-Tolylsulfonamino; Alkoxycarbonyloxy wie Ethoxycarbonyloxy, Benzyloxycarbonyloxy; Aryloxycarbonyloxy wie Phenoxycarbonyloxy; Alkyl- oder Arylthio wie Dodecylthio, 1-Carboxydodecylthio, Phenylthio, 2-Butoxy-5-t-octylphenylthio, Tetrazolylthio; Carbamoylamino wie N-Methylcarbamoylamino, N-Phenylcarbamoylamino; Imidazolyl, Pyrazolyl, Triazolyl, Tetrazolyl, 1,2-Dihydro-2-oxo-1-pyridyl; Imido wie Succinimido, Hydantoinyl; Arylazo wie Phenylazo, 4-Methoxyphenylazo.

Des weiteren kann X z.B. mit dem Kuppler der Formel (C-III) die Bis-Verbindung der Formel

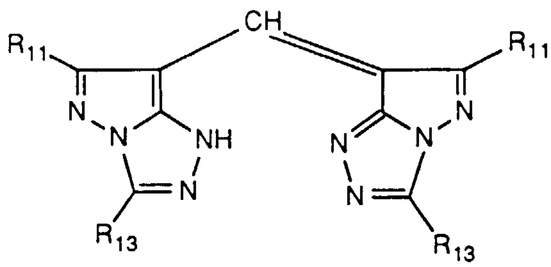

(C-IIIa)

und auch entsprechende Bis-Verbindungen mit den Kupplern der Formeln (C-I), (C-II) und (C-IV) bilden.

X kann auch photographisch wirksame Gruppen wie Entwicklungsinhibitoren oder -beschleuniger enthalten. Vorzugsweise ist X jedoch Halogen oder einer der genannten Alkoxy-, Aryloxy-, Alkyl- oder Arylthioreste oder ein 5- oder 6-gliedriger stickstoffhaltiger Ring, der über ein Stickstoffatom an das Pyrazoloazolsystem gebunden ist

Vorzugsweise werden Pyrazoloazolkuppler mit den Verbindungen der Formel (1) stabilisiert.

Die Verbindungen der Formel (1) können auf übliche Weise in Schichten photographischer Materialien eingearbeitet werden. Diese Schichten können reine Bindemittelschichten, z.B. Gelatineschichten, oder silberhalogenidhaltige Schichten sein, wobei als Silberhalogenid die üblichen Halogenide wie Chlorid, Bromid und Jodid sowie Mischungen dieser Halogenide in Frage kommen. Die Schichten können auch sonstige in photographischen Materialien übliche Komponenten enthalten wie Antischleiermittel, Filterfarbstoffe, optische Aufheller, UV-Absorber und übliche organische Stabilisatoren wie sterisch gehinderte Amine und Phenole. Neben Magentakuppler enthalten die Schichten gegebenenfalls auch Mischungen von Kupplern, z.B. von Magentakupplern oder Magenta-, Cyan- und Gelbkupplern.

Insbesondere im Hinblick auf die Stabilisierung von Magentabildfarbstoffen hat sich die Verwendung einer Kombination von Verbindungen der Formel (1) mit bekannten Lichtschutzmitteln vom Hydrochinon- und insbesondere Hydrochinonäthertyp bewährt. Diese Verbindungen liegen vorzugsweise in derselben Schicht vor wie die Verbindungen der Formel (1).

Solche Hydrochinon-Verbindungen sind in folgenden Patentschriften näher beschrieben: US-A-2,360,290, 2,336,327, 2,403,721, 2,418,613, 2,675,314, 2,701,197, 2,710,801, 2,732,300, 2,728,659, 2,735,765, 2,704,713, 2,937,086, 2,816,028, 3,582,333, 3,637,393, 3,700,453, 3,960,570, 3,935,016, 3,930,866, 4,065,435, 3,982,944, 4,232,114, 4,121,939, 4,175,968, 4,179,293, 3,591,381, 3,573,052, 4,279,990, 4,429,031, 4,346,165, 4,360,589, 4,346,167, 4,385,111, 4,416,978, 4,430,425, 4,277,558, 4,489,155, 4,504,572, 4,559,297, FR-A-885,982; GB-A-891,158, 1,156,167, 1,363,921, 2,022,274, 2,066,975, 2,071,348, 2,081,463, 2,117,526, 2,156,091; DE-A-2,408,168, 2,726,283, 2,639,930, 2,901,520, 3,308,766, 3,320,483, 3,323,699; DD-A-216,476, 214,468, 214,469, EP-A-84290, 110,214, 115,305, 124,915, 124,877, 144,288, 147,747, 178,165, 161,577; JP-A-75/33733, 75/21249, 77/128,130, 77/146,234, 79/70036, 79/133,131, 81/83742, 81/87040, 81/109,345, 83/134,628, 82/22237, 82/112,749, 83/17431, 83/21249, 84/75249, 84/149,348, 84/182,785, 84/180,557, 84/189,342, 84/228,249, 84/101,650, 79/24019, 79/25823, 86/48856, 86/48857, 86/27539, 86/6652, 86/72040, 87/11455, 87/62157, sowie in Research Disclosure 79/17901, 79/17905, 79/18813, 83/22827 und 84/24014.

Hydrochinonäther sind in folgenden Patentschriften näher beschrieben:

US-A 3,285,937, 3,432,300, 3,519,429, 3,476,772, 3,591,381, 3,573,052, 3,574,627, 3,573,050, 3,698,909,

3,764,337, 3,930,866, 4,113,488, 4,015,990, 4,113,495, 4,120,723, 4,155,765, 4,159,910, 4,178,184, 4,138,259, 4,174,220, 4,148,656, 4,207,111, 4,254,216, 4,314,011, 4,273,864, 4,264,720, 4,279,990, 4,332,886, 4,436,165, 4,360,589, 4,416,978, 4,385,111, 4,459,015, 4,559,297, 4,616,082, 4,631,252; GB-A 1,347,556, 1,366,441, 1,547,392, 1,557,237, 2, 135,788; DE-A 3,214,567; DD-214,469, EP-A 161,577, 167,762, 164,130, 176,845; JP-A 76/123,642, 77/35633, 77/147,433, 78/126, 78/10430, 78/53321, 79/24019, 79/25823, 79/48537, 79/44521, 79/56833, 79/70036, 79/70830, 79/73032, 79/95233, 79/145,530, 80/21004, 80/50244, 80/52057, 80/70840, 80/139,383, 81/30125, 81/151,936, 82/34552, 82/68833, 82/204,036, 82/204,037, 83/134,634, 83/207,039, 84/60434, 84/101,650, 84/87450, 84/149,348, 84/182,785, 86/72040, 87/11455, 87/62157, 87/63149, 86/2151, 86/6652, 86/48855 sowie in Research Disclosure 78/17051.

Beispiele besonders geeigneter Lichtschutzmittel, die in Kombination mit den Verbindungen der Formel (1) verwendbar sind, entsprechen den Formeln

(A) 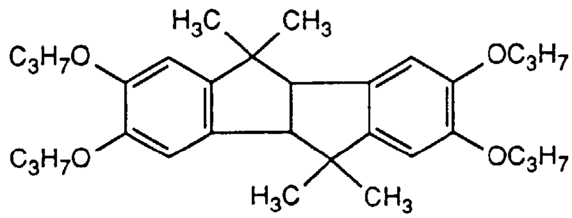 ,

(B) 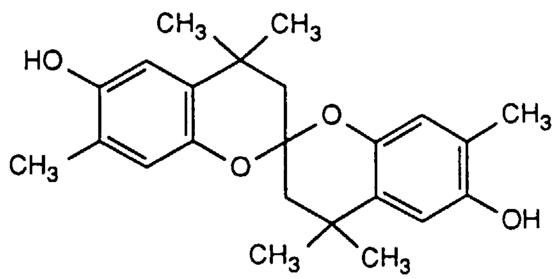 ,

(C) 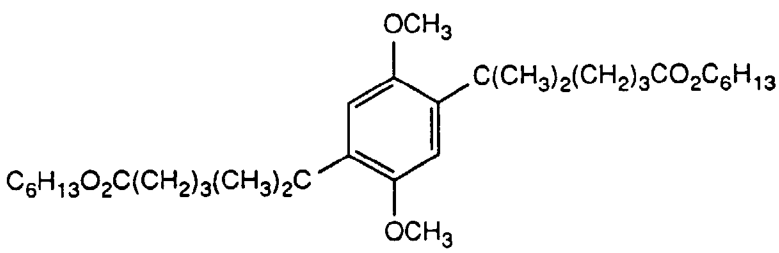 ,

(D) 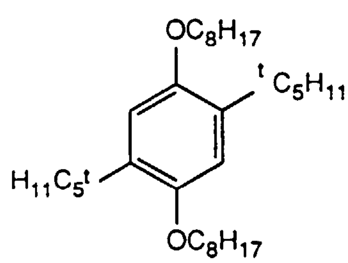 ,

(E) 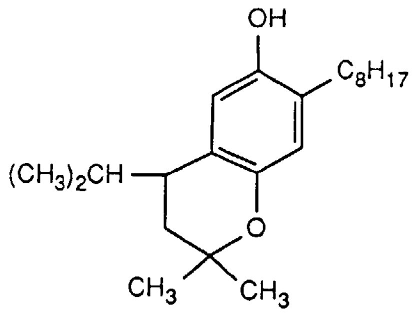

und

(F) 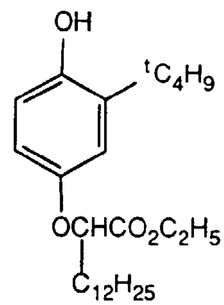 .

Gegenstand der vorliegenden Erfindung sind auch die neuen Verbindungen der Formel

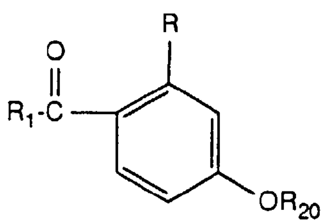 (1a)

worin R Wasserstoff oder Hydroxyl, $R_1$ Alkyl oder Alkoxy mit je 1 bis 18 Kohlenstoffatomen ist, oder $R_1$ $-NR_4R_5$, worin $R_4$ und $R_5$ unabhängig voneinander Wasserstoff oder Alkyl mit 1 bis 18 Kohlenstoffatomen sind, oder $R_1$ ein Rest der Formel

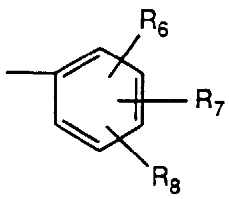 ,

worin $R_6$, $R_7$ und $R_8$ unabhängig voneinander Wasserstoff, Hydroxyl oder Alkyl mit 1 bis 8 Kohlenstoffatomen sind, oder $R_1$ ein Rest der Formel

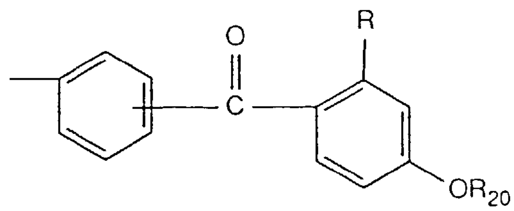

ist, worin R die angegebene Bedeutung hat, und $R_{20}$ ein Rest der Formel

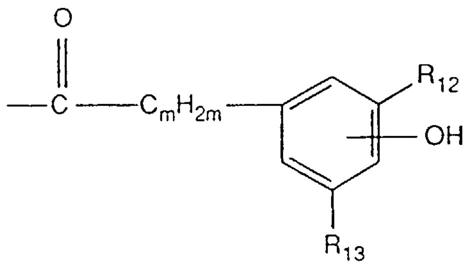

ist, worin $R_{12}$ und $R_{13}$ unabhängig voneinander Wasserstoff, Alkyl mit je 1 bis 8 Kohlenstoffatomen, Cyclopentyl, und m 1 bis 6 ist, oder $R_{20}$ -CO-Alkenyl mit 2 bis 18 Kohlenstoffatomen im Alkenylteil oder, wenn R Wasserstoff ist, -CO-Alkyl mit 1 bis 18 Kohlenstoffatomen im Alkylteil ist, wobei die Verbindung der Formel

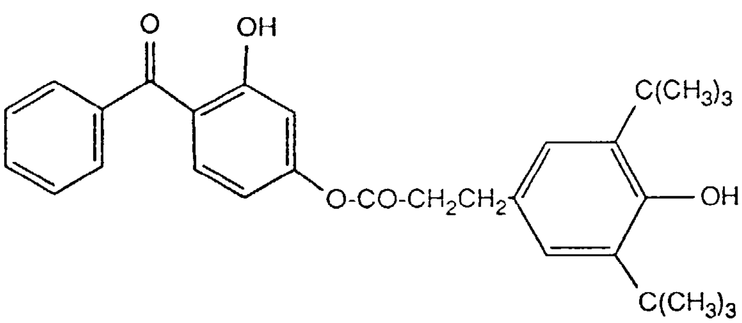

(18)

ausgeschlossen ist, die in MATERIE PLASTICHE ELASTOMER 1, Januar 1975, Italy, Seiten 41-44; J.I.Temchin et al : "Dependance of the efficiency of photostabilizers for polypropylene upon their chemical structure", in der Tabelle 1 auf der Seite 42 beschrieben ist.

Beispiele für die in den Verbindungen der Formel (1a) verwendeten Substituenten sind an entsprechender Stelle in den Erläuterungen der Substituenten der Verbindungen der Formel (1) zu finden.

Auch diese Verbindungen der Formel (1a) und insbesondere solche, worin $R_{12}$ und $R_{13}$ unabhängig voneinander Wasserstoff oder Alkyl mit je 1 bis 4 Kohlenstoffatomen sind und m 2 ist, eignen sich gut zur Stabilisierung von Magentakupplern in photographischen Materialien. Eine weitere Gruppe besonders bevorzugter Stabilisatoren sind solche Verbindungen der Formel (1a), worin $R_{20}$-CO-Alkenyl mit 12 bis 18 Kohlenstoffatomen im Alkenylteil ist, sowie jene Verbindungen, worin $R_1$ Alkyl oder Alkoxy mit je 1 bis 4 Kohlenstoffatomen oder ein Rest der Formel

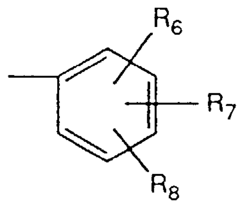

ist,

worin $R_6$, $R_7$ und $R_8$ unabhängig voneinander Wasserstoff, Hydroxyl oder Alkyl mit je 1 bis 4 Kohlenstoffatomen sind. Sie können insbesondere zur Stabilisierung von Magentakupplern vom genannten Pyrazoloazol-Typ verwendet werden. In Kombination mit den oben erwähnten Lichtschutzmitteln vom Hydrochinon- und Hydrochinonäthertyp stellen die Verbindungen der Formel (1a) wirksame Stabilisatoren für Magentabildfarbstoffe dar.

Die Verbindungen der Formel (1) sowie die erfindungsgemässen Verbindungen der Formel (1a) lassen sich auf an sich bekannte Weise herstellen. Beispielsweise wird durch Friedel-Crafts-Acylierung minels $AlCl_3$ oder $ZnCl_2$ ein Phenol der Formel

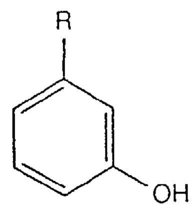

mit $R_1COCl$ acyliert und durch weitere Acylierung der Substituent $R_{20}$ in das Molekül eingeführt.

Die folgenden Beispiele erläutern die Erfindung weiter. Prozentangaben beziehen sich auf das Gewicht.

Herstellungsbeispiele

Beispiel 1: Herstellung von 3,5-Di-tert.butyl-2',4,4'-trihydroxy-benzophenon 107,5 g 3,5-Di-tert.butyl-4-hydroxybenzoesäurechlorid und 48,4 g Resorcin werden in 400 ml Nitrobenzol suspendiert und auf 50°C erwärmt. Die braune Lösung wird auf 0°C abgekühlt. Unter gutem Rühren werden 58,7 g Aluminiumchlorid während 45 Minuten portionenweise zugegeben. Das Reaktionsgemisch wird während 18 Stunden langsam auf 20°C gebracht. Nun werden weitere 4 Stunden bei 50°C gerührt, anschliessend wird auf Raumtemperatur abgekühlt und auf 500 ml Salzsäure/Eis gegossen. Die dicke gelbe Suspension wird mit Aether extrahiert. Die Aetherphase wird mit 500 ml Natriumhydroxyd (15 %) extrahiert. Die basische Lösung wird dreimal mit je 250 ml Aether gewaschen, mit 15%iger Salzsäure auf einen pH-Wert von 1-2 gebracht und mit Aether extrahiert. Die Aetherextrakte werden vereinigt, getrocknet und unter vermindertem Druck eingedampft.

Man erhält 124,4 g 3,5-Di-tert.butyl-2',4,4'-trihydroxy-benzophenon, das nach Umkristallisation aus Methanol einen Schmelzpunkt von 192-193°C besitzt.

Beispiel 2: Herstellung der Verbindung der Formel (2) 12,0 g 3,5-Di-tert.butyl-2',4,4'-trihydroxybenzophenon und 3,9 g Triäthylamin werden in 20 ml Tetrahydrofuran und 40 ml Toluol gelöst. Die orange-beige Lösung wird auf 0°C abgekühlt. Unter gutem Rühren wird eine Lösung aus 11,2 g 3,5-Di-tert.butyl-4-hydroxyphenylpropionsäurechlorid in 40 ml Toluol tropfenweise zwischen 0 und 5°C während 1 Stunde zugegeben und anschliessend 2 Tage bei Raumtemperatur gerührt. Das Reaktionsgemisch wird in 700 ml Eiswasser gegossen und die Phasen getrennt. Die wässrige Phase wird mit Toluol extrahiert, die Toluolphasen vereinigt, gewaschen, getrocknet und unter vermindertem Druck eingeengt. Das beige Oel wird über Kieselgel chromatographiert und aus Petroläther (40-60°C)/Essigester umkristallisiert. Man erhält 21,0 g 4'-[3,5-Di-tert.butyl-4-hydroxyphenylpropionyloxy]-3,5-di-tert,butyl-2',4-dihydroxybenzophenon mit einem Schmelzpunkt von 145-147°C.

Beispiel 3: Verfährt man wie in Beispiel 2 beschrieben und setzt die entsprechende Menge 3,5-Di-tert.butyl-4-hydroxybenzoesäurechlorid ein, so erhält man 4'-[3,5-Di-tert.butyl-4-hydroxybenzoyloxy]-3,5-di-tert.butyl-2',4-dihydroxybenzophenon mit einem Schmelzpunkt von 120-122°C.

Beispiel 4: Verfährt man wie in Beispiel 2 beschrieben und setzt die entsprechende Menge 3-tert.Butyl-4-hydroxy-5-isopropylphenylpropionsäurechlorid ein, so erhält man 4'-[3-tert.Butyl-4-hydroxy-5-isopropylphenylpropionyloxy]-3,5-di-tert.butyl-2',4-dihydroxybenzophenon (Verbindung Nr. 3) mit einem Schmelzpunkt von 154-157°C.

<u>Beispiel 5</u>: Verfährt man wie in Beispiel 2, beschrieben und setzt die entsprechende Menge 3,5-Di-tert.butyl-4-hydroxyphenyl-2-methylpropionsäurechlorid ein, so erhält man 4'-[3,5-Di-tert.butyl-4-hydroxyphenyl-2-methylpropionyloxy]-3,5-di-tert.butyl-2',4-dihydroxybenzophenon mit einem Schmelzpunkt von 163-165°C.

<u>Beispiel 6</u>: Verfährt man wie in Beispiel 2 beschrieben und setzt die entsprechende Menge Essigsäurechlorid ein, so erhält man 4-Acetoxy-3,5-di-tert.butyl-2',4-dihydroxybenzophenon mit einem Schmelzpunkt 182-186°C.

<u>Beispiel 7</u>: Verfährt man wie in Beispiel 1 beschrieben und setzt die entsprechende Menge 2,4-Dihydroxybenzophenon ein, so erhält man 4-[3,5-Di-tert.butyl-4-hydroxy-benzoloxy]-2',hydroxybenzophenon mit einem Schmelzpunkt von 122-124°C.

<u>Anwendungsbeispiele</u>

<u>Beispiele 8-22</u>:0,044 g des Magentakupplers der Formel

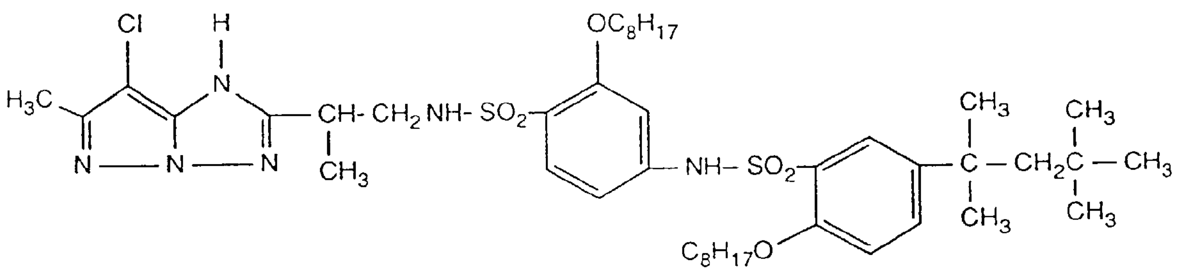

und 0,0155 g eines in der nachfolgenden Tabelle angegebenen Stabilisators werden in 2 ml eines Gemisches von Trikresylphosphat und Ethylacetat (1,1 g/100 ml) gelöst.

Zu 1,0 ml dieser Lösung gibt man 9,0 ml einer 2,3%-igen wässrigen Gelatinelösung, die auf einen pH-Wert von 6,5 eingestellt ist und 0,436 g/l des Netzmittels der Formel

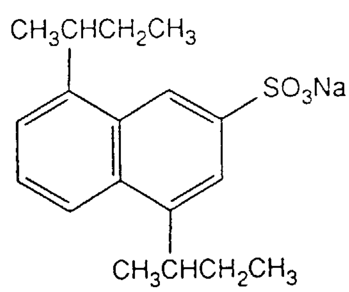

Anschliessend wird die Lösung mit Ultraschall während 3 Minuten emulgiert.

Zu 5 ml der so erhaltenen Kuppleremulsion gibt man 2 ml einer Silberbromidemulsion mit einem Silbergehalt von 3 g/l sowie 1 ml einer 0,7%-igen wässrigenLösung des Härters der Formel

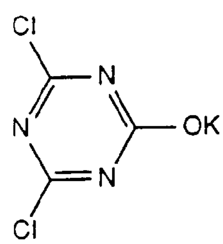

Man vergiesst die so erhaltene Mischung auf ein kunststoffbeschichtetes Papier (13 x18 cm). Nach einer Härtungszeit von 7 Tagen wird die Probe hinter einem Stufenkeil mit 125 Lux·s belichtet und anschliessend im Ektaprint 2®-Prozess

(Kodak) verarbeitet.

Der so erhaltene Magentakeil wird in einem Klimaschrank bei 75°C und 60 % rel. Luftfeuchtigkeit 28 Tage lang gelagert. Danach wird die Klimavergilbung, die ein Mass für die Dunkelstabilität photographischer Bilder ist, durch Bestimmung der $D_{min}$(Blau) - Werte (Macbeth Densitometer TR 924®) am Anfang und am Ende der Behandlung ermittelt:

$$\text{Klimavergilbung} = [D_{min}(\text{Blau})]_{28}-[D_{min}(\text{Blau})_{o}.$$

Als Vergleich wird eine Probe ohne Stabilisator denselben Bedingungen unterworfen. Die Ergebnisse sind in der folgenden Tabelle aufgeführt.

Tabelle 1:

| Beispiel | Stabilisator der Formel | Klimavergilbung |
|---|---|---|
| 8 | ohne | 0,25 |
| 9 | (2) | 0,16 |
| 10 | (3) | 0,16 |
| 11 | (22) | 0,18 |
| 12 | (23) | 0,16 |
| 13 | (21) | 0,17 |
| 14 | (24) | 0,18 |
| 15 | (28) | 0,18 |
| 16 | (29) | 0,18 |
| 17 | (30) | 0,19 |
| 18 | (27) | 0,13 |
| 19 | (31) | 0,18 |
| 20 | (32) | 0,15 |
| 21 | (33) | 0,16 |
| 22 | (10) | 0,16 |

Die erfindungsgemäss verwendeten Stabilisatoren zeigen eine gute Stabilisierung des Magentakupplers in den verwendeten Proben.

Beispiel 23: Man stellt Magentakeile gemäss Beispiele 8-22 her, aber mit dem Unterschied, dass statt 0,0155 g eines Stabilisators der Formel (2) nur 0,0078 g eingesetzt werden bzw. 0,0155 g des Lichtschutzmittels der Formel (A) bzw. 0,0078 g Stabilisator der Formel (2) zusammen mit 0,0155 g Lichtschutzmittel der Formel (A). Diese Keile werden wie in den Beispielen 8-22 gezeigt im Klimaschrank behandelt, zusätzlich aber auch in einem Atlas Weather-Ometer hinter einem UV-Filter (Kodak 2c) mit einer 2500 W-Xenonlampe mit 30 k Joule/$cm^2$ bestrahlt.

Es wird der während der Behandlung eingetretene Farbdichteverlust (%) beim Absorptionsmaximum des Magentabildfarbstoffes mit einem Densitometer (TR 924 A der Fa. Macbeth) gemessen und in der nachfolgenden Tabelle wiedergegeben. Je kleiner der Dichteverlust, desto höher ist die Lichtschutzwirkung.

Tabelle 2:

| zusätzliches Lichtschutzmittel | Stabilisator der Formel | Farbdichteverlust (%) | Klimavergilbung |
|---|---|---|---|
| - | - | 87 | 0,25 |
| (A) | - | 21 | 0,24 |
| - | (2) | 86 | 0,17 |
| (A) | (2) | 17 | 0,18 |

**Patentansprüche**

1. Verfahren zum Stabilisieren von Magentakupplern und den entsprechenden Bildfarbstoffen in photographischen Materialien, dadurch gekennzeichnet, dass in die Magentakuppler enthaltende Schicht oder in eine an diese angrenzende Schicht mindestens eine Verbindung der Formel

(1) 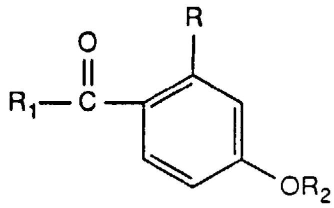

eingearbeitet wird, worin
R Wasserstoff oder Hydroxyl,
$R_1$ Alkyl oder Alkoxy mit je 1 bis 18 Kohlenstoffatomen, $-NR_4R_5$, worin $R_4$ und $R_5$ unabhängig voneinander Wasserstoff oder Alkyl mit 1 bis 18 Kohlenstoffatomen sind, oder ein Rest der Formel

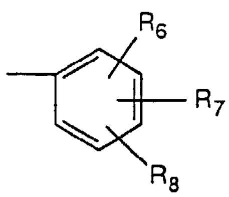

ist, worin $R_6$, $R_7$ und $R_8$ unabhängig voneinander Wasserstoff, Hydroxyl oder Alkyl mit je 1 bis 8 Kohlenstoffatomen sind, oder $R_1$ ein Rest der Formel

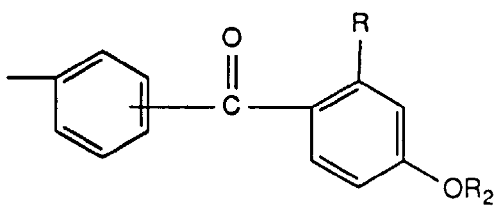

ist, worin R die angegebene Bedeutung hat, und
$R_2$ $-COR_{11}$ oder $-SO_2R_{11}$, worin $R_{11}$ Alkyl mit 1 bis 18 Kohlenstoffatomen, Alkenyl mit 2 bis 18 Kohlenstoffatomen, Phenylalkyl mit 1 bis 4 Kohlenstoffatomen im Alkylteil oder ein Rest der Formel

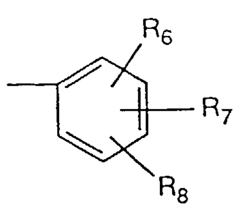 ,

ist, worin $R_6$, $R_7$ und $R_8$ die angegebenen Bedeutungen haben, oder $R_2$ ein Rest der Formel

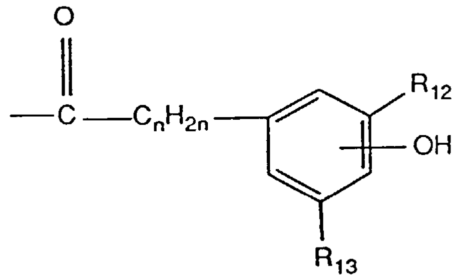

oder

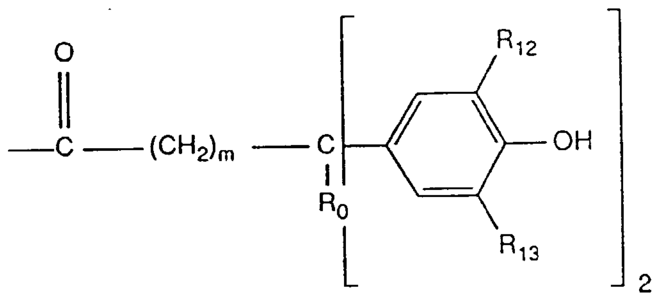

ist, worin
m 1 bis 6 und $R_0$ Wasserstoff oder Methyl ist, und
n 0 bis 14 ist, und $R_{12}$ und $R_{13}$ unabhängig voneinander Wasserstoff, Alkyl mit je 1 bis 8 Kohlenstoffatomen, Cyclopentyl, Cyclohexyl, Alkylphenyl mit 1 bis 8 Kohlenstoffatmen im Alkylteil oder Phenyl sind.

**2.** Verfahren nach Anspruch 1, worin in der Verbindung der Formel (1) $R_1$ Alkyl oder Alkoxy mit je 1 bis 8 Kohlenstoffatomen oder ein Rest der Formel

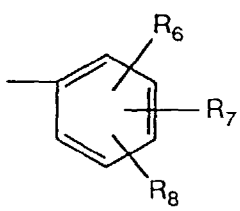 oder 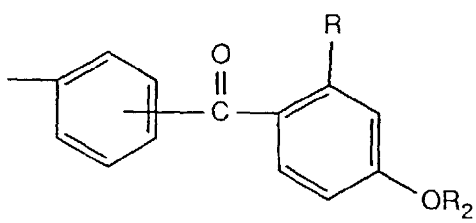

ist, worin R, $R_2$, $R_6$, $R_7$ und $R_8$ die in Anspruch 1 angegebenen Bedeutungen haben.

**3.** Verfahren nach Anspruch 2, worin in der Verbindung der Formel (1) $R_1$ Alkyl oder Alkoxy mit je 1 bis 8 Kohlenstoffatomen oder ein Rest der Formel

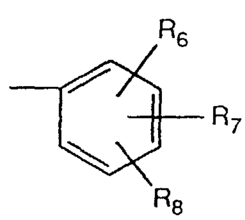

ist, worin $R_6$, $R_7$ und $R_8$ unabhängig voneinander Wasserstoff, Hydroxyl oder Alkyl mit 1 bis 4 Kohlenstoffatomen sind, $R_2$ -$COR_{11}$, worin $R_{11}$ Alkyl mit 1 bis 18 Kohlenstoffatomen ist, oder ein Rest der Formel

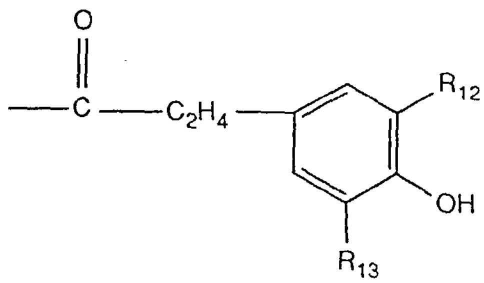

ist, worin $R_{12}$ und $R_{13}$ unabhängig voneinander Wasserstoff oder Alkyl mit 1 bis 4 Kohlenstoffatomen sind.

**4.** Verfahren nach Anspruch 3, worin in der Verbindung der Formel (1) $R_1$ Alkyl oder Alkoxy mit je 1 bis 4 Kohlenstoffatomen oder ein Rest der Formel

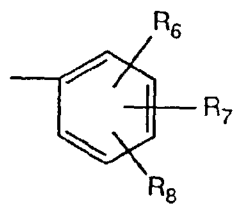

ist, worin $R_6$, $R_7$ und $R_8$ unabhängig voneinander Wasserstoff, Hydroxyl oder Alkyl mit 1 bis 4 Kohlenstoffatomen sind, und $R_2$ -$COR_{11}$, worin $R_{11}$ Alkyl mit 12 bis 18 Kohlenstoffatomen ist, oder ein Rest der Formel

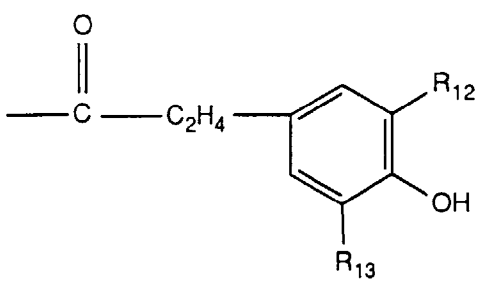

ist, worin $R_{12}$ und $R_{13}$ Alkyl mit je 1 bis 4 Kohlenstoffatomen sind.

**5.** Verfahren nach Anspruch 1, dadurch gekennzeichnet, dass der Magentakuppler eine Pyrazoloazolverbindung ist.

**6.** Verfahren nach Anspruch 1, dadurch gekennzeichnet, dass die Verbindung der Formel (1) zusammen mit einem Lichtschutzmittel vom Hydrochinon- oder Hydrochinonäthertyp eingearbeitet wird.

**7.** Verbindungen der Formel

(1a) 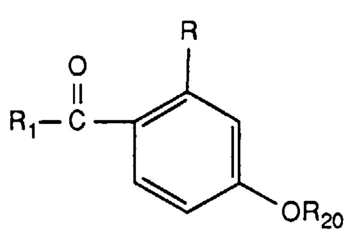 ,

worin R Wasserstoff oder Hydroxyl, $R_1$ Alkyl oder Alkoxy mit je 1 bis 18 Kohlenstoffatomen ist, oder $R_1$ $-NR_4R_5$, worin $R_4$ und $R_5$ unabhängig voneinander Wasserstoff oder Alkyl mit 1 bis 18 Kohlenstoffatomen sind, oder $R_1$ ein Rest der Formel

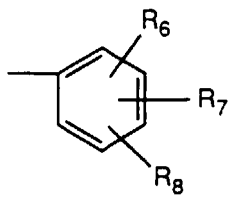

,

worin $R_6$, $R_7$ und $R_8$ unabhängig voneinander Wasserstoff, Hydroxyl oder Alkyl mit 1 bis 8 Kohlenstoffatomen sind, oder $R_1$ ein Rest der Formel

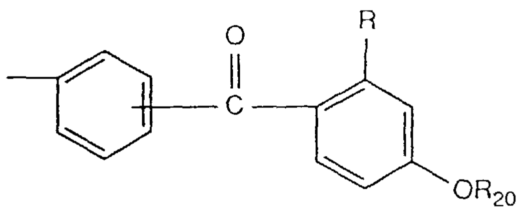

ist, worin R die angegebene Bedeutung hat, und $R_{20}$ ein Rest der Formel

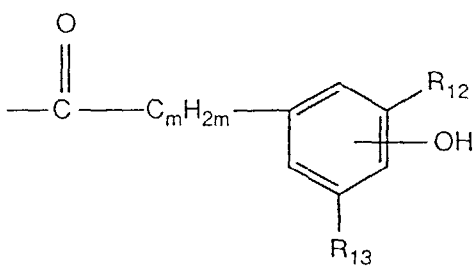

ist, worin $R_{12}$ und $R_{13}$ unabhängig voneinander Wasserstoff, Alkyl mit je 1 bis 8 Kohlenstoffatomen, Cyclopentyl, und m 1 bis 6 ist, oder $R_{20}$ -CO-Alkenyl mit 2 bis 18 Kohlenstoffatomen im Alkenylteil oder, wenn R Wasserstoff ist, -CO-Alkyl mit 1 bis 18 Kohlenstoffatomen im Alkylteil ist, wobei die Verbindung der Formel

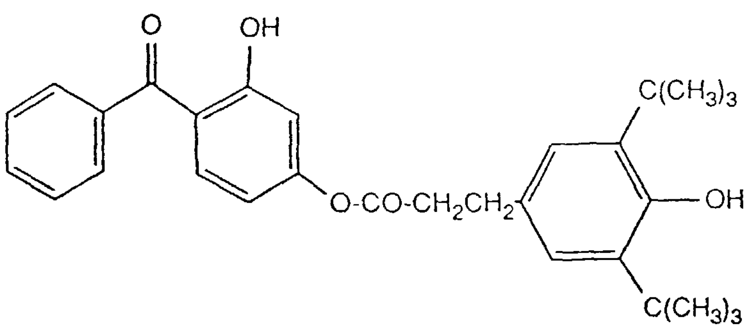

(18)

ausgeschlossen ist.

8. Verbindungen nach Anspruch 7, worin $R_{12}$ und $R_{13}$ unabhängig voneinander Wasserstoff oder Alkyl mit 1 bis 4

Kohlenstoffatmen sind, und m 2 ist.

**9.** Verbindungen nach Anspruch 7, worin $R_{20}$ -CO-Alkenyl mit 12 bis 18 Kohlenstoffatomen im Alkenylteil ist.

**10.** Verbindungen nach Anspruch 7, worin $R_1$ Alkyl oder Alkoxy mit je 1 bis 4 Kohlenstoffatomen oder ein Rest der Formel

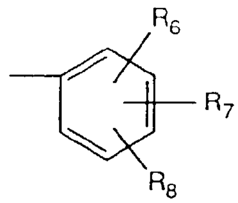

ist, worin $R_6$, $R_7$ und $R_8$ unabhängig voneinander Wasserstoff, Hydroxyl oder Alkyl mit je 1 bis 4 Kohlenstoffatomen sind.

**11.** Photographisches Material, enthaltend mindestens eine in Anspruch 1 definierte Verbindung der Formel (1), worin die Verbindung der Formel (1) in einer Magentakuppler enthaltenden Schicht oder in einer an diese Schicht angrenzenden Schicht enthalten ist.

## Claims

**1.** A process for stabilising magenta couplers and the corresponding image dyes in photographic materials, which comprises incorporating at least one compound of the formula

(1) 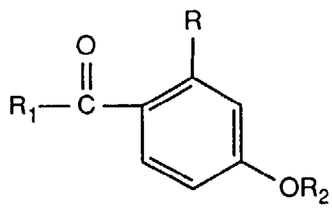

in which R is hydrogen or hydroxyl, $R_1$ is alkyl or alkoxy, in each case having 1 to 18 carbon atoms, $-NR_4R_5$ in which $R_4$ and $R_5$, independently of one another, are hydrogen or alkyl having 1 to 18 carbon atoms, or $R_1$ is a radical of the formula

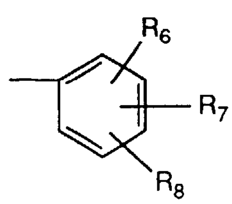

in which $R_6$, $R_7$ and $R_8$, independently of one another, are hydrogen, hydroxyl or alkyl, in each case having 1 to 8 carbon atoms, or $R_1$ is a radical of the formula

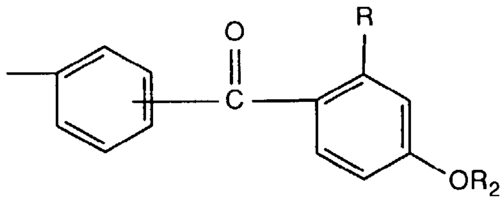

in which R is as defined above, and $R_2$ is $-COR_{11}$ or $-SO_2R_{11}$ in which $R_{11}$ is alkyl having 1 to 18 carbon atoms, alkenyl having 2 to 18 carbon atoms, phenylalkyl having 1 to 4 carbon atoms in the alkyl moiety or a radical of the formula

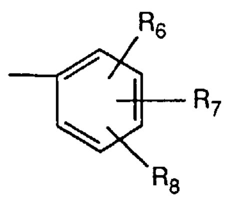

in which $R_6$, $R_7$ and $R_8$ are as defined above, or $R_2$ is a radical of the formula

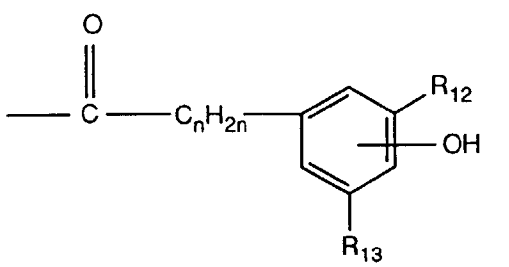

or

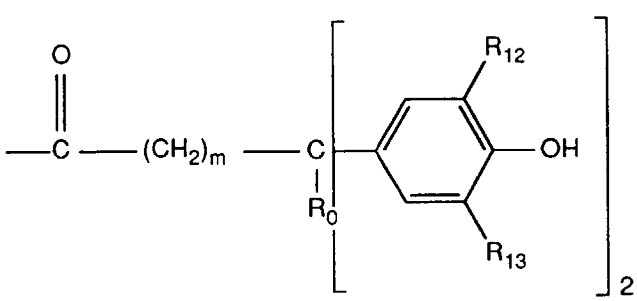

in which m is 1 to 6, and $R_0$ is hydrogen or methyl, and n is 0 to 14, and $R_{12}$ and $R_{13}$, independently of one another, are hydrogen, alkyl, in each case having 1 to 8 carbon atoms, cyclopentyl, cyclohexyl, alkylphenyl having 1 to 8 carbon atoms in the alkyl moiety, or phenyl,
into the layer containing magenta couplers or into a layer adjacent thereto.

**2.** A process according to claim 1, in which, in the compound of the formula (1), $R_1$ is alkyl or alkoxy in each case having 1 to 8 carbon atoms, or is a radical of the formula

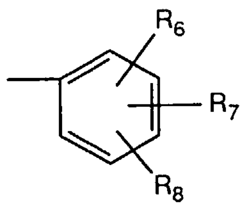 or 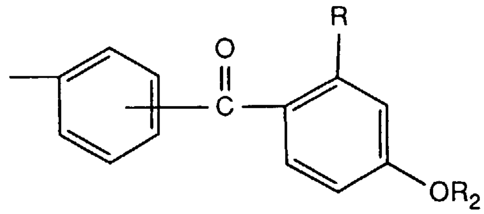

in which R, $R_2$, $R_6$, $R_7$ and $R_8$ are as defined in claim 1.

3. A process according to claim 2, in which, in the compound of the formula (1), $R_1$ is alkyl or alkoxy in each case having 1 to 8 carbon atoms, or is a radical of the formula

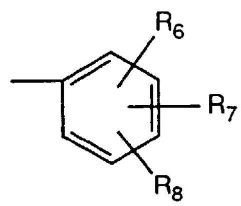

in which $R_6$, $R_7$ and $R_8$, independently of one another, are hydrogen, hydroxyl or alkyl having 1 to 4 carbon atoms, $R_2$ is $-COR_{11}$ in which $R_{11}$ is alkyl having 1 to 18 carbon atoms, or a radical of the formula

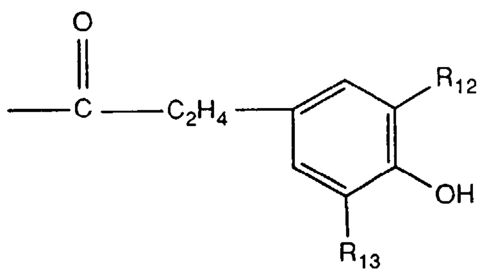

in which $R_{12}$ and $R_{13}$, independently of one another, are hydrogen or alkyl having 1 to 4 carbon atoms.

4. A process according to claim 3, in which, in the compound of the formula (1), $R_1$ is alkyl or alkoxy in each case having 1 to 4 carbon atoms, or is a radical of the formula

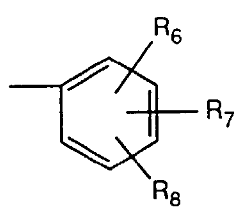

in which $R_6$, $R_7$ and $R_8$, independently of one another, are hydrogen, hydroxyl or alkyl having 1 to 4 carbon atoms, and $R_2$ is $-COR_{11}$ in which $R_{11}$ is alkyl having 12 to 18 carbon atoms, or a radical of the formula

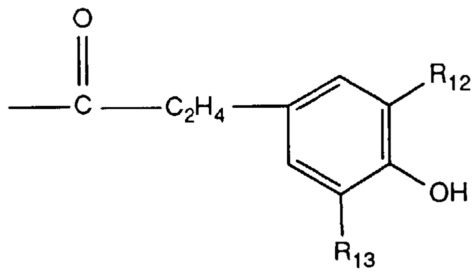

in which $R_{12}$ and $R_{13}$ are alkyl in each case having 1 to 4 carbon atoms.

**5.** A process according to claim 1, wherein the magenta coupler is a pyrazoloazole compound.

**6.** A process according to claim 1, wherein the compound of the formula (1) is incorporated together with a light screen of the hydroquinone or hydroquinone ether type.

**7.** A compound of the formula

(1a) 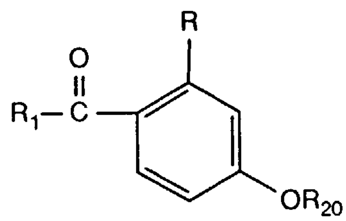

in which R is hydrogen or hydroxyl, $R_1$ is alkyl or alkoxy in each case having 1 to 18 carbon atoms, or $-NR_4R_5$ in which $R_4$ and $R_5$, independently of one another, are hydrogen or alkyl having 1 to 18 carbon atoms, or $R_1$ is a radical of the formula

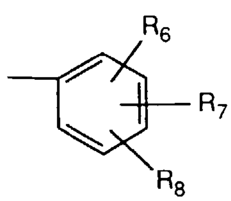

in which $R_6$, $R_7$ and $R_8$, independently of one another, are hydrogen, hydroxyl or alkyl having 1 to 8 carbon atoms, or $R_1$ is a radical of the formula

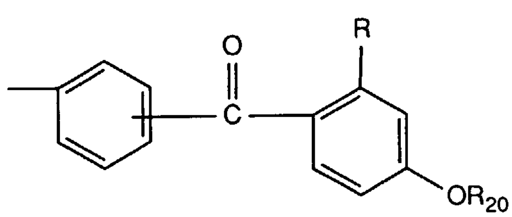

in which R is as defined above, and $R_{20}$ is a radical of the formula

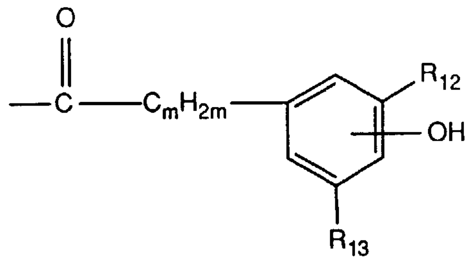

in which $R_{12}$ and $R_{13}$, independently of one another, are hydrogen, alkyl in each case having 1 to 8 carbon atoms, or cyclopentyl, and m is 1 to 6, or $R_{20}$ is -CO-alkenyl having 2 to 18 carbon atoms in the alkenyl moiety or, if R is hydrogen, -CO-alkyl having 1 to 18 carbon atoms in the alkyl moiety, with the exception of the compound of the formula (18).

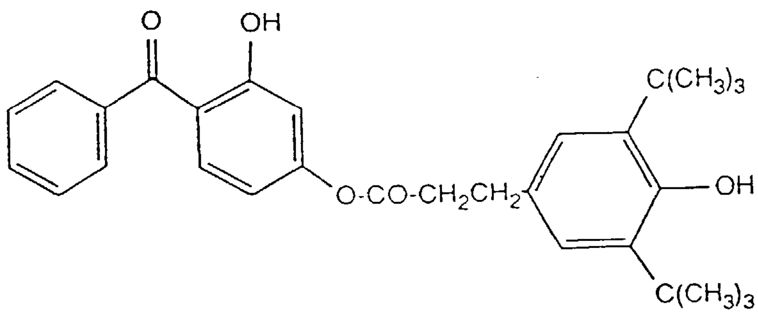

(18)

**8.** A compound according to claim 7, in which $R_{12}$ and $R_{13}$, independently of one another, are hydrogen or alkyl having 1 to 4 carbon atoms, and m is 2.

**9.** A compound according to claim 7, in which $R_{20}$ is -CO-alkenyl having 12 to 18 carbon atoms in the alkenyl moiety.

**10.** A compound according to claim 7, in which $R_1$ is alkyl or alkoxy in each case having 1 to 4 carbon atoms, or a radical of the formula

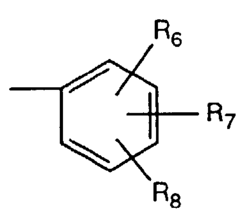

in which $R_6$, $R_7$ and $R_8$, independently of one another, are hydrogen, hydroxyl or alkyl in each case having 1 to 4 carbon atoms.

**11.** A photographic material comprising at least one compound of the formula (1) defined in claim 1, in which the compound of the formula (1) is present in a layer containing magenta couplers or in a layer adjacent thereto.

## Revendications

1. Procédé pour la stabilisation de copulants magenta et des matières colorantes d'images correspondantes dans des matières photographiques, caractérisé en ce que l'on incorpore dans la couche contenant le copulant magenta ou dans une couche limitrophe de celle-ci au moins un composé de formule

(1) 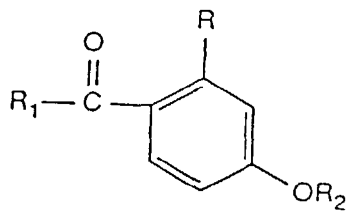

où

R représente l'hydrogène ou hydroxyle,
$R_1$ représente alkyle ou alcoxy chacun avec 1 à 18 atomes de carbone, $-NR_4R_5$, où $R_4$ et $R_5$, indépendamment l'un de l'autre, représentent l'hydrogène ou alkyle avec 1 à 18 atomes de carbone, ou
$R_1$ représente un radical de formule

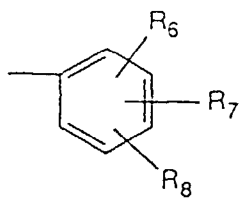

où $R_6$, $R_7$ et $R_8$, indépendamment les uns des autres, représentent l'hydrogène, hydroxyle ou alkyle chacun avec 1 à 8 atomes de carbone, ou $R_1$ représente un radical de formule

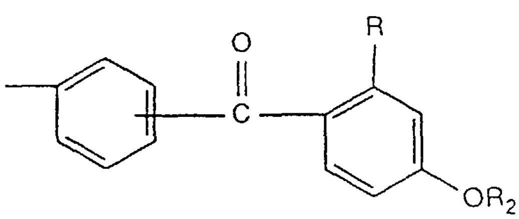

où R a la signification donnée, et
$R_2$ représente $-COR_{11}$ ou $-SO_2R_{11}$, où $R_{11}$ représente alkyle avec 1 à 18 atomes de carbone, alcényle avec 2 à 18 atomes de carbone, phénylalkyle avec 1 à 4 atomes de carbone dans la partie alkyle ou un radical de formule

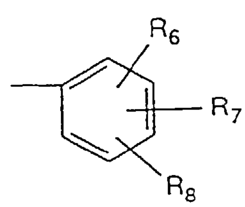

où $R_6$, $R_7$ et $R_8$ ont les significations données ou $R_2$ représente un radical de formule

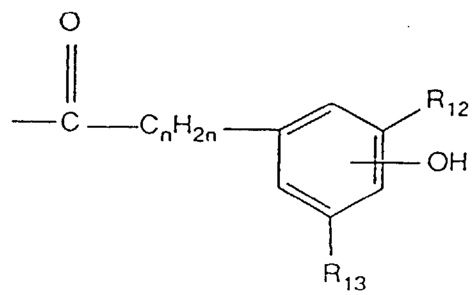

ou

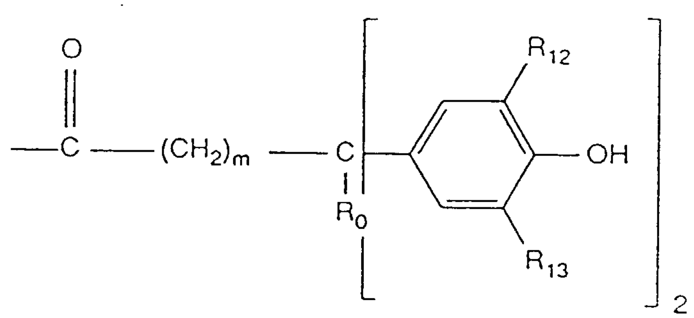

ou

m va de 1 à 6 et $R_0$ représente l'hydrogène ou méthyle,

et

n va de 0 à 14, et $R_{12}$ et $R_{13}$, indépendamment l'un de l'autre, représentent l'hydrogène, alkyle chacun avec 1 à 8 atomes de carbone, cyclopentyle, cyclohexyle, alkylphényle avec 1 à 8 atomes de carbone dans la partie alkyle, ou représentent phényle.

**2.** Procédé selon la revendication 1 où, dans le composé de formule (1), $R_1$ représente alkyle ou alcoxy chacun avec 1 à 8 atomes de carbone ou un radical de formule

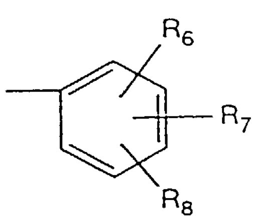

ou

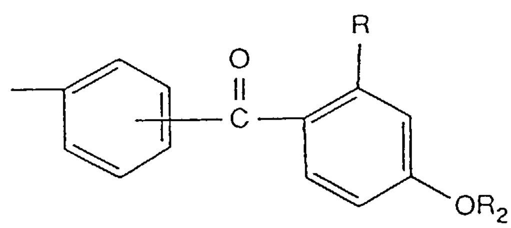

où R, $R_2$, $R_6$, $R_7$ et $R_8$ ont la signification donnée dans la revendication 1.

**3.** Procédé selon la revendication 2 où, dans le composé de formule (1), $R_1$ représente alkyle ou alcoxy chacun avec 1 à 8 atomes de carbone ou un radical de formule

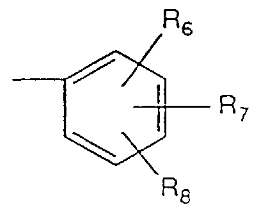

où $R_6$, $R_7$ et $R_8$, indépendamment les uns des autres, représentent l'hydrogène, hydroxyle ou alkyle avec 1 à 4 atomes de carbone, $R_2$ représente $-COR_{11}$, où $R_{11}$ est alkyle avec 1 à 18 atomes de carbone, ou un radical de formule

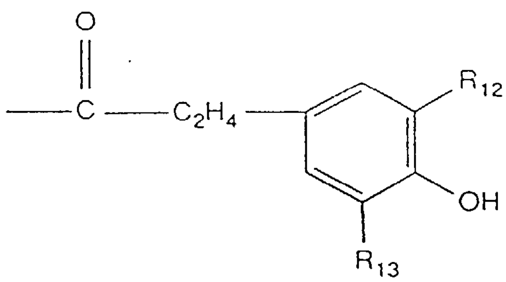

dans laquelle $R_{12}$ et $R_{13}$, indépendamment l'un de l'autre, représentent l'hydrogène ou alkyle avec 1 à 4 atomes de carbone.

**4.** Procédé selon la revendication 3 où, dans le composé de formule (1), $R_1$ représente alkyle ou alcoxy chacun avec 1 à 4 atomes de carbone, ou un radical de formule

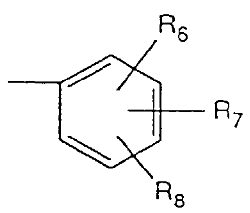

dans laquelle $R_6$, $R_7$ et $R_8$, indépendamment l'un de l'autre, représentent l'hydrogène, hydroxyle ou alkyle avec 1 à 4 atomes de carbone, et $R_2$ représente $-COR_{11}$, où $R_{11}$ représente alkyle avec 12 à 18 atomes de carbone ou un radical de formule

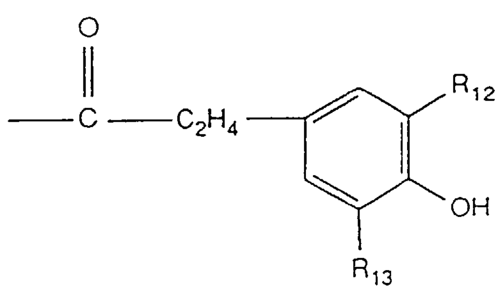

dans laquelle $R_{12}$ et $R_{13}$ représentent alkyle chacun avec 1 à 4 atomes de carbone.

**5.** Procédé selon la revendication 1, caractérisé en ce que le copulant magenta est un composé de pyrazoloazole.

**6.** Procédé selon la revendication 1, caractérisé en ce que le composé de formule (1) est incorporé ensemble avec un agent photoprotecteur de type hydroquinone ou éther d'hydroquinone.

**7.** Composés de formule

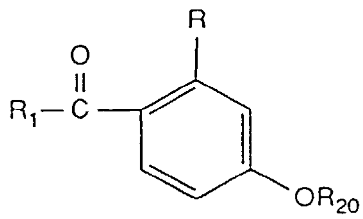

(1a)

dans laquelle R représente l'hydrogène ou hydroxy, $R_1$ représente alkyle ou alcoxy chacun avec 1 à 18 atomes de carbone, où $R_1$ représente $-NR_4R_5$, dans laquelle $R_4$ et $R_5$, indépendamment l'un de l'autre, sont l'hydrogène ou alkyle avec 1 à 18 atomes de carbone, ou $R_1$ est un radical de formule

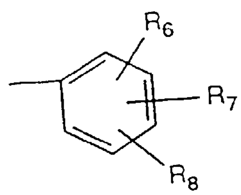

dans laquelle $R_6$, $R_7$ et $R_8$, indépendamment les uns des autres, représentent l'hydrogène, hydroxyle ou alkyle avec 1 à 8 atomes de carbone, ou $R_1$ représente un radical de formule

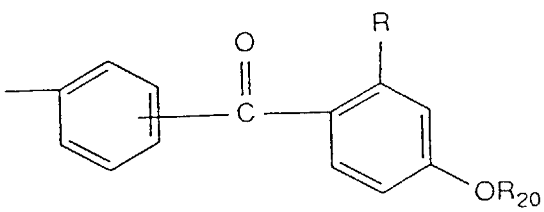

dans laquelle R a la signification donnée et $R_{20}$ représente un radical de formule

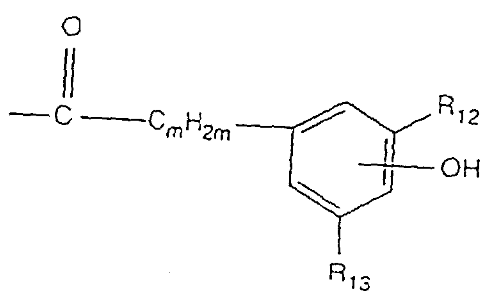

dans laquelle $R_{12}$ et $R_{13}$, indépendamment l'un de l'autre, représentent l'hydrogène, alkyle chacun avec 1 à 8 atomes de carbone, cyclopentyle et m va de 1 à 6, ou $R_{20}$ représente -CO-alcényle avec 2 à 18 atomes de carbone dans la partie alcényle ou, lorsque R représente l'hydrogène, -CO-alkyle avec 1 à 18 atomes de carbone dans la partie alkyle, le composé de formule

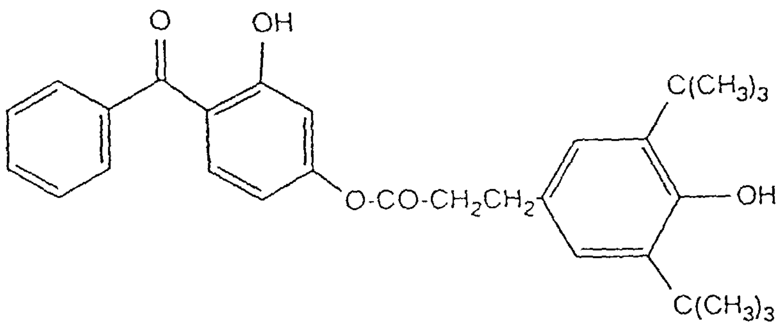

(18)

étant exclu.

**8.** Composés selon la revendication 7, où $R_{12}$ et $R_{13}$, indépendamment l'un de l'autre, représentent l'hydrogène ou alkyle avec 1 à 4 atomes de carbone et m vaut 2.

**9.** Composés selon la revendication 7, où $R_{20}$ représente -CO-alcényle avec 12 à 18 atomes de carbone dans la partie alcényle.

**10.** Composés selon la revendication 7, où $R_1$ représente alkyle ou alcoxy chacun avec 1 à 4 atomes de carbone ou un radical de formule

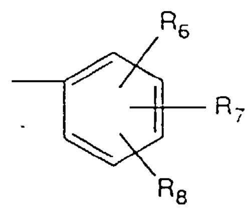

dans laquelle $R_6$, $R_7$ et $R_8$, indépendamment l'un de l'autre, représentent l'hydrogène, hydroxyle ou alkyle chacun avec 1 à 4 atomes de carbone.

**11.** Matière photographique contenant au moins un composé de formule (1) défini dans la revendication 1, où le composé de formule (1) est contenu dans une couche contenant le copulant magenta ou dans une couche limitrophe de celle-ci.